# EUROPÄISCHE PATENTSCHRIFT

(11) **EP 4 254 020 B1**
(45) Veröffentlichungstag und Bekanntmachung des Hinweises auf die Patenterteilung: **25.02.2026**
(21) Anmeldenummer: 23183798.0
(22) Anmeldetag: 08.09.2020
(51) Int. Cl.: G01N 21/3504, G01N 21/17, G01V 8/20

(54) **SYSTEM UND VERFAHREN ZUR ÜBERWACHUNG EINES LUFTRAUMES FÜR EIN AUSGEDEHNTES GELÄNDE**
SYSTEM AND METHOD FOR MONITORING AN AIR SPACE FOR AN EXTENSIVE SITE
SYSTÈME ET PROCÉDÉ DE SURVEILLANCE D'UN ESPACE D'AIR POUR UN SITE ÉTENDU

(30) Priorität: 09.09.2019 DE 102019124092
(43) Veröffentlichungstag der Anmeldung: 04.10.2023
(62) Teilanmeldung aus: 20771249.8
(73) Patentinhaber: GRANDPERSPECTIVE GmbH, 14532 Kleinmachnow (DE)
(72) Erfinder: Braun, René, 10999 Berlin (DE); Maas, Peter, 04177 Leipzig (DE)
(74) Vertreter: Cohausz & Florack

(56) Entgegenhaltungen:
- WO-A1-2016/029305
- JP-A- 2011 204 118
- US-A- 4 795 253
- US-A1- 2013 208 262
- US-A1- 2017 097 274
- US-A1- 2018 292 286
- US-A1- 2019 170 900
- US-B2- 10 267 729
- BONOW G ET AL: "Gas leak localization in industrial environments using a TDLAS-based remote gas sensor and autonomous mobile robot with the Tri-Max method", 2013 IEEE INTERNATIONAL CONFERENCE ON ROBOTICS AND AUTOMATION (ICRA); 6-10 MAY 2013; KARLSRUHE, GERMANY, IEEE, US, 6 May 2013 (2013-05-06), pages 987 - 992, XP032505894, ISSN: 1050-4729, ISBN: 978-1-4673-5641-1, [retrieved on 20131013], DOI: 10.1109/ICRA.2013.6630693

## Beschreibung

Die Erfindung betrifft ein System zur Überwachung eines Luftraumes für ein Gelände wie z.B. industrielle chemische Anlagen, Häfen oder kritische Verkehrsinfrastruktur. Insbesondere dient die Erfindung der Detektion und Lokalisation von Gasleckagen und potenziell gefährlichen Gaswolken.

Auf den genannten Geländen können durch Undichtigkeiten oder Fehlfunktionen von Anlagen Gase austreten, die für die Umwelt schädlich sind, zu Unfällen mit weitreichenden Folgen führen können und Gefahren für Gesundheit und Leben der sich auf dem Gelände aufhaltenden Personen darstellen können.

Eine gängige Überwachung eines Geländes erfolgt mit chemo-elektrischen Sensoren, die jeweils nur für ein Gas oder eine geringe Anzahl von chemischen Gasen empfindlich sind. Zudem müssen die Sensoren dort angeordnet sein, wo die Gase in der Umgebungsluft vorhanden sind. Daher ist die Anzahl der einzusetzenden Sensoren hoch.

Weiterhin ist die Anwendung von Fourier-Transform-Infrarot-Spektrometern (FTIR) bekannt, die in kurzen Zeitabständen die Zusammensetzung des beobachteten Raumwinkels oberhalb des Geländes auf das Vorhandensein verschiedener chemischer Gase bestimmen können. Damit kann das ungewollte Austreten von Gasen detektiert werden und mittels Triangulation der Ort ermittelt werden. Jedoch mangelt es den bekannten Verfahren an einer guten räumlichen Auflösung und somit einer zuverlässigen Lokalisation der Austrittsstelle, insbesondere bei einer Beeinträchtigung der Sichtverhältnisse durch topographische Hindernisse.

Ein aus dem Stand der Technik bekanntes FTIR-Spektrometer dient dabei als optischer Sensor. Das Spektrometer selbst ist ortsfest ausgebildet und dessen Infrarot-Optik ist in einem nach oben gerichteten Winkel ausgerichtet. Mit einem schwenkbaren Spiegel wird das Umgebungslicht auf die Infrarot-Optik gerichtet und somit die Umgebung gescannt. Der Überwachungsbereich ist dabei allerdings auf einen geringen Raumwinkelbereich beschränkt.

Die EP 2 088 409 B1 offenbart ein abbildendes Spektrometer für die Fernerkundung zum Erzeugen eines spektral aufgelösten Bildes, jedoch ohne eine räumliche Auflösung des gemessenen Geländes.

Die US 4,795,253 A offenbart das kontinuierliche Überwachen von in der Umgebung von Produktionseinrichtungen vorhandenen gasförmigen Materialien, bei dem drei Detektoren die unmittelbare Umgebung oberhalb einer Emissionsquelle durch Triangulation ermitteln.

Die US 2019/170900 A1 offenbart ein Hub-and-Spoke System mit einer zentralen und ortsfest installieren Spektrometer-/Detektoreinheit und mehreren Reflektoren, die auf einem zu überwachenden Gelände verteilt sind.

Die WO 2016/029305 A1 beschreibt eine einzelne Gasdetektionsvorrichtung, die auf einer Drohne installiert werden kann.

Daher liegt der vorliegenden Erfindung das technische Problem zugrunde, ein System und ein Verfahren zur Überwachung eines Luftraumes für ein ausgedehntes Gelände zu verbessern.

Das zuvor aufgeführte technische Problem wird erfindungsgemäß durch ein System der nachfolgend beschriebenen Art und Weise gelöst.

Ein System zur Überwachung eines Luftraumes für ein ausgedehntes Gelände ist ausgerüstet mit mindestens zwei optischen Sensoren mit einem passiven Fourier-Transform-Infrarotspektrometer und mit einem Server zur Auswertung der Messdaten und zur Steuerung der mindestens zwei optischen Sensoren, wobei jeder optischer Sensor einen einstellbaren Überwachungsbereich aufweist und wobei sich die Überwachungsbereiche der mindestens zwei optische Sensoren zumindest abschnittsweise überlappen und wobei der Server eingerichtet, die optischen Sensoren im Regelfall zu einem automatischen Scannen der Überwachungsbereiche anzusteuern, wobei der Server den Messdaten jeweils einen Raumwinkel anhand der Positionsdaten des optischen Sensors zuordnet, aus den Messdaten der optischen Sensoren für jeden Raumwinkel die spektrale Intensitätsverteilung der empfangenen IR-Strahlung abzuleiten und mittels Korrelation der Intensitätsverteilung mit bekannten Gasspektren, mindestens einen Zielstoff zu identifizieren, im Ereignisfall, wenn ein erster optischer Sensor in einem ersten Raumwinkel einen Zielstoff identifiziert, mindestens einen weiteren optischen Sensor anzusteuern, den Überlappungsbereich mit dem Überwachungsbereich des ersten optischen Sensors zu scannen, aus den Messdaten des mindestens einen weiteren optischen Sensors mindestens einen weiteren Raumwinkel mit einem Infrarotsignal des Zielstoffes zu identifizieren und aus den Raumwinkelinformationen des ersten Raumwinkels und des mindestens einen weiteren Raumwinkels die Koordinaten des Überlappungsbereiches mit erhöhter Konzentration des Zielstoffes zu ermitteln. Das System ist dadurch gekennzeichnet, dass der Überwachungsbereich jedes optischen Sensors durch die Topographie und/oder die Bebauung des Geländes aufgrund von Abschattungen abhängig vom Raumwinkel unterschiedliche Messradien aufweist und der Überwachungsbereich jedes optischen Sensors durch den Raumwinkelbereich und die zugehörigen Messradien festgelegt ist und dass die Messsignale des mindestens einen weiteren optischen Sensors in Raumrichtungen mit zu geringem Messradius nicht in die Auswertung einbezogen werden.

Das System wird so verstanden, dass es insbesondere, aber nicht nur bei größeren Systemen mit einer Mehrzahl von optischen Sensoren ausreicht, wenn sich die Überwachungsbereiche von jeweils zumindest zwei optischen Sensoren (2; 2a, 2b, 2c) zumindest abschnittsweise überlappen. Somit werden notwendiger Weise paarweise, jedoch nicht von allen Paaren von optischen Sensoren eine Überlappung der Überwachungsbereiche gefordert. Ein Gesamtsystem kann somit auch als eine Kombination von mehreren Systemen verstanden werden, bei denen sich die Überwachungsbereiche aller der mindestens zwei optischen Sensoren zumindest abschnittsweise überlappen.

Der genannte Server kann dabei auch als Auswerteeinheit oder als Auswerte- und Steuerungseinheit bezeichnet werden.

Der Server kann vorzugsweise auch mehrere voneinander getrennte Untereinheiten aufweisen, die bevorzugt jeweils in einem optischen Sensor integriert oder zumindest benachbart angeordnet sind. Beispielsweise kann der Sensor je eine Untereinheit aufweisen, die zusammen den Server bzw. die Auswerteeinheit bilden. Ferner können verschiedene Aufgaben des Servers durch Untereinheiten übernommen werden. So kann beispielsweise eine erste Untereinheit des Servers die Messdaten auswerten und eine zweite Untereinheit einen oder mehrere optische Sensoren steuern.

In einer bevorzugten Ausführungsform ist der Server als zentrale Steuereinheit ausgebildet, die zwei oder mehr optische Sensoren steuert und/oder die Messdaten von zwei oder mehr optischen Sensoren auswertet. Besonders bevorzugt steuert die zentrale Steuereinheit alle Sensoren und/oder wertet die Messdaten aller optischen Sensoren des Systems aus. Der Server kann vorzugsweise mit großem räumlichen Abstand zu den optischen Sensoren angeordnet sein. Ferner kann der Server auch ein wechselnder Server eines Servernetzwerks sein. Beispielsweise können das Auswerten der Messdaten und/oder das Steuern der optischen Sensoren auch unter Verwendung einer Cloud erfolgen, die von einem oder mehreren Servern bereitgestellt wird.

Innerhalb eines Raumwinkels entspricht der Messradius dabei der maximalen Entfernung, bis zu der der optische Sensor ein Messsignal aufnehmen kann. Der Messradius kann der maximale Messradius sein, den der optische Sensor realisieren kann. Der Messradius kann aber auch aufgrund einer Abschattung durch ein Hindernis in seiner räumlichen Erstreckung begrenzt, also kürzer sein. Der optische Sensor kann also in diesem Raumwinkel keine Messdaten für den Bereich hinter dem Hindernis erzeugen. Die Messradien pro Raumwinkel werden bei der Installation des Systems für die optischen Sensoren anhand der bekannten Topographie und/oder Bebauung des Geländes bestimmt und festgelegt.

Die Messradien der optischen Sensoren sind bevorzugt abhängig vom Raumwinkel einstellbar. Der Messradius wird dabei für jeden Raumwinkel ausgehend vom optischen Sensor bis zur maximalen Entfernung oder bis zu einem eine Abschattung verursachenden Hindernis eingestellt. Vorzugsweise ist das System dazu ausgebildet, die Messradien für die Raumwinkel, vorzugsweise aus zwei- und/oder dreidimensionalen Kartendaten, automatisch zu definieren. Beispielsweise und bevorzugt kann das System dazu eingerichtet sein, die Messradien unter Verwendung eines digitalen Zwillings und/oder eines vereinfachten dreidimensionalen Modells des zu überwachenden Geländes zu definieren. Ferner bevorzugt ist das System dazu eingerichtet, mittels einer Kamera des optischen Sensors Hindernisinformationen zu erfassen, und die Messradien unter Verwendung der Hindernisinformationen zu definieren. Die Hindernisinformationen korrespondieren bevorzugt zu optischen Hindernissen, die vorzugsweise entlang einer optischen Achse des Sensors angeordnet sind. Besonders bevorzugt ist das System eingerichtet, die Messradien unter Verwendung von Hindernisinformationen mehrerer optischer Sensoren zu definieren.

Unter einem Raumwinkel wird weiterhin ein räumlicher Raumwinkelbereich verstanden, den der optische Sensor auflösen kann. Der Raumwinkelbereich eines Raumwinkels, also die räumliche Auflösung des optischen Sensors kann innerhalb des Systems einstellbar sein. Für jeden Raumwinkel des Raumwinkelbereichs entspricht somit der zugehörige Messradius der maximalen Entfernung, bis zu der der optische Sensor ein Messsignal aufnehmen kann. Für alle Raumwinkel des Raumwinkelbereichs kann der optische Sensor optische Signale empfangen und korrespondierende Messsignale bereitstellen.

Somit werden also Messsignale eines optischen Sensors nur dann verwendet, wenn der zugehörige Messradius des optischen Sensors bis zu einem erforderlichen Überlappungsbereich mit dem Überwachungsbereichs eines anderen optischen Sensors reicht.

Der Server ist vorzugsweise für eine der vorbeschriebenen Funktionen auch dann eingerichtet, wenn nur eine Komponente des Servers für eine der vorbeschriebenen Funktionen eingerichtet ist. Beispielsweise kann der Server als Auswerteeinheit dazu eingerichtet sein, die optischen Sensoren im Regelfall zu einem automatischen Scannen der Überwachungsbereiche anzusteuern und/oder den Messdaten jeweils einen Raumwinkel anhand der Positionsdaten des optischen Sensors zuzuordnen. Wenn also ein erster optischer Sensor einen Zielstoff identifiziert, steuert der Server einen weiteren Sensor an, um einen Überlappungsbereich des Überwachungsbereichs des ersten Sensors mit dem Überwachungsbereich des weiteren Sensors zu scannen. Hierdurch kann das System vorzugsweise eine von dem ersten optischen Sensor erfolgte Identifikation des Zielstoffs verifizieren.

Des Weiteren ist der Server eingerichtet, die Messsignale des mindestens einen weiteren optischen Sensors, die nicht in die Auswertung einbezogen worden sind, durch mathematische Interpolation, insbesondere eine gewichtete Interpolation von räumlich angrenzenden Messsignalen zu ersetzen. Angrenzende Messsignale sind dabei Messsignale, die für Raumwinkel bereitgestellt werden, die im Raumwinkelbereich räumlich benachbart zu demjenigen Raumwinkel mit zu geringem Messradius liegen.

Weiterhin sind mindestens drei optische Sensoren vorgesehen und der Server ist eingerichtet, im Ereignisfall zumindest einen weiteren optischen Sensor für eine Ansteuerung auszuwählen, dessen Überwachungsbereich eine maximale Überlappung mit dem Überwachungsbereich des ersten optischen Sensors aufweist.

Vorzugsweise ist der Server eingerichtet, einen Raumwinkel des ersten optischen Sensors, für den der Zielstoff identifiziert wird, mit den Überwachungsbereichen der weiteren Sensoren abzugleichen und einen weiteren optischen Sensor für eine Ansteuerung auszuwählen, dessen Überwachungsbereich den Überwachungsbereich des ersten Sensors für denjenigen Raumwinkel überlappt, für den der erste optische Sensor den Zielstoff identifiziert.

In bevorzugter Weise ist der Server eingerichtet, einen ausgewählten optischen Sensor nicht einzusetzen, wenn die Position des ausgewählten optischen Sensors in Richtung des Raumwinkelbereichs des ersten optischen Sensors mit nachgewiesenem Zielstoff liegt. In einem solchen Fall wird vorzugsweise der nächstbeste optische Sensor ausgewählt, der eine möglichst große Überlappung mit dem Überwachungsbereich des ersten optischen Sensors aufweist.

So kann erreicht werden, dass die optische Achse des ersten Sensors für den Raumwinkel, für den der Zielstoff identifiziert wird, und die optische Achse des weiteren optischen Sensors, der zum Scannen des Überlappungsbereichs angesteuert wird, winklig zueinander angeordnet sind. Aus den Raumwinkeln der optischen Sensoren, für die der Zielstoff identifiziert wird, kann dann vorzugsweise mittels Triangulation eine Position des Zielstoffs ermittelt werden.

Weiterhin ist es vorteilhaft, wenn der Server eingerichtet ist, aus den Messdaten der optischen Sensoren die Säulendichten des Zielstoffes zu ermitteln, wobei die Säulendichte das mathematische Produkt aus Konzentration des Gases (gemessen in ppm) und der räumlichen Länge der Gaswolke (gemessen in m) ist und wobei der Server eingerichtet ist, die Koordinaten des Überlappungsbereiches der höchsten Säulendichten verschiedener optischer Sensoren zu ermitteln.

In einer bevorzugten Ausgestaltung ist das System und ist insbesondere der Server eingerichtet, im Ereignisfall mittels des ersten optischen Sensors eine Säulendichte des Zielstoffs im ersten Raumwinkel zu bestimmen und eine Säulendichte des Zielstoffs in an den ersten Raumwinkel angrenzenden Raumwinkeln zu bestimmen. An den ersten Raumwinkel angrenzende Raumwinkel schließen vorzugsweise kontinuierlich an den ersten Raumwinkel an. Es ist aber auch bevorzugt, dass ein angrenzender Raumwinkel einen vordefinierten Winkelabstand zum ersten Raumwinkel hat. Wenn beispielsweise der erste Raumwinkel einen Wert von 45° aufweist, können angrenzende Raumwinkel beispielsweise Werte von 44° und 46° aufweisen.

Gemäß einer bevorzugten Weiterbildung ist das System und insbesondere der Server eingerichtet, aus dem ersten Raumwinkel und den angrenzenden Raumwinkeln denjenigen Raumwinkel mit der höchsten Säulendichte des Zielstoffs zu identifizieren und mittels des ersten optischen Sensors eine Säulendichte des Zielstoffs in einem an den Raumwinkel mit der höchsten Säulendichte angrenzenden Raumwinkel zu bestimmen. Das System ist also eingerichtet, die im ersten Schritt bestimmten und den Raumwinkeln zugeordneten Säulendichten des Zielstoffs zu vergleichen und denjenigen Raumwinkel zu ermitteln, unter dem die höchste Säulendichte des Zielstoffs bestimmt wird. Anschließend wird zu einem an diesen Raumwinkel der höchsten Zielstoffsäulendichte angrenzenden Raumwinkel erneut die Säulendichte des Zielstoffs bestimmt. Als angrenzender Raumwinkel wird vorzugsweise ein Raumwinkel mit unbekannter Säulendichte des Zielstoffs gewählt. Wird beispielsweise wie obenstehend beschrieben unter einem Raumwinkel von 44° die höchste Säulendichte des Zielstoffs bestimmt, wird anschließend die Säulendichte des Zielstoffs für einen Raumwinkel mit einem Wert von 43° ermittelt.

Vorzugsweise ist das System und ist insbesondere der Server eingerichtet, den Raumwinkel des Überwachungsbereichs des ersten optischen Sensors zu bestimmen, der im Ereignisfall die höchste Säulendichte des Zielstoffs aufweist. Hierfür ist das System eingerichtet, die zuvor beschriebenen Schritte auszuführen, bis derjenige Raumwinkel ermittelt ist, für den die Säulendichte des Zielstoffs maximal ist. Wird beispielsweise unter 43° und 45° eine geringere Säulendichte des Zielstoffs ermittelt, als für einen Raumwinkel von 44°, ist der Raumwinkel mit dem Wert von 44° derjenige Raumwinkel des Überwachungsbereichs, der die höchste Säulendichte des Zielstoffs aufweist. Das System kann aber auch dazu ausgebildet sein, mehrere lokale Maxima der Säulendichte des Zielstoffs im Überwachungsbereich zu ermitteln.

Bevorzugt ist das das System und insbesondere der Server eingerichtet, im Ereignisfall einen zu den Raumwinkeln des ersten optischen Sensors korrespondierenden ersten Konzentrationsgradienten des Zielstoffs zu bestimmen. Der Konzentrationsgradient ist ein Maß für die Änderung der Zielstoffsäulendichte je diskretem Raumwinkelschritt.

In einer bevorzugten Weiterbildung ist das System und insbesondere der Server eingerichtet, im Ereignisfall aus dem zu Raumwinkeln des ersten optischen Sensors korrespondierenden ersten Konzentrationsgradienten und einem analogen weiteren Konzentrationsgradienten eines weiteren optischen Sensors einen mehrdimensionalen Konzentrationsgradienten des Zielstoffs zu bestimmen. Vorzugsweise werden also zu den Raumwinkeln einer Mehrzahl von Sensoren Konzentrationsgradienten bestimmt und vom System kombiniert, um den mehrdimensionalen Konzentrationsgradienten zu bestimmen. Beispielsweise kann der mehrdimensionale Konzentrationsgradient ein Maß für die Änderung der Zielstoffsäulendichte in einer ersten Raumrichtung und in einer zur ersten Raumrichtung senkrechten Raumrichtung sein.

Vorzugsweise ist das System und ist insbesondere der Server dazu eingerichtet, die ermittelte Säulendichte des Zielstoffs mit einem Zielstoffgrenzwert zu vergleichen.

Besonders bevorzugt ist das System und insbesondere der Server dazu eingerichtet, einen Alarm auszulösen, wenn die ermittelte Säulendichte des Zielstoffs den Zielstoffgrenzwert übersteigt, vorzugsweise für einen vordefinierten Zeitraum übersteigt.

Das Erfassen von Messdaten, welche die Säulendichten der jeweiligen Raumwinkel repräsentieren, kann auch vor einem Ermitteln der jeweiligen Säulendichten aus diesen Messdaten erfolgen. Beispielsweise kann ein optischer Sensor Messdaten für mehrere Raumwinkel erfassen und das Ermitteln der an den jeweiligen Raumwinkeln vorliegenden Säulendichten bzw. des Raumwinkels, der die höchste Säulendichte des Zielstoffs aufweist, erfolgt anschließend durch den Server in seiner Funktion als Auswerteeinheit. Vorzugsweise können das Erfassen von Messdaten und das Ermitteln der Säulendichten und/oder der höchsten Säulendichte auch simultan oder teilweise simultan erfolgen.

In bevorzugter Weise ist der Server weiterhin eingerichtet, im Ereignisfall die optischen Sensoren für Messungen mit einer erhöhten Ortsabtastung anzusteuern. Dabei wird die Scangeschwindigkeit verlangsamt und gegebenenfalls die Messdauer für einen Raumwinkel vergrößert, um ein besseres Signal-zu-Rauschen-Verhältnis zu erlangen.

Vorzugsweise ist der Server weiterhin eingerichtet, die Koordinaten des Überlappungsbereiches der höchsten Säulendichten verschiedener optischer Sensoren zu ermitteln und die Koordinaten mit einer Kartendarstellung zu verknüpfen und eine zweidimensionale Darstellung des Ereignisses zu erstellen und/oder die Koordinaten mit den Bildern einer Videokamera zu verknüpfen und eine visuelle Darstellung des Ereignisses zu erstellen. Dadurch wird eine schnelle und eindeutige Information für den Betreiber des Systems geschaffen.

Des Weiteren kann mindestens eine aktive Infrarotstrahlungsquelle vorgesehen sein und ein optischer Sensor nimmt das Infrarotlicht entlang einer definierten Messstrecke auf. Dadurch wird die Messgenauigkeit des Systems entlang dieser Messstrecken erhöht.

Der Begriff "der Server ist eingerichtet" bedeutet, dass der Server als Computer mit einer Computerumgebung ausgebildet ist, wobei geeignete Microchips, Speicherchips bzw. Speichermedien sowie Schnittstellen zu externen, gegebenenfalls auch entfernten Geräten vorgesehen sind und wobei mindestens ein Computerprogramm vorhanden ist, um die beschriebenen Funktionen technisch zu realisieren.

In weiter bevorzugter Weise kann bei dem zuvor beschriebenen System mindestens ein stationärer Detektor vorgesehen sein, wobei der Server eingerichtet ist, ein Ausgangssignal des mindestens einen stationären Detektors als auslösendes Signal für den Einsatz der optischen Sensoren im Raumbereich des stationären Sensors zu verwenden. Somit kann im Ereignisfall die Identifikation des Zielstoffs unter Verwendung der stationären Messdaten getriggert werden. Befindet sich der stationäre Detektor im Bereich einer Leckage innerhalb des Systems, kann durch das Messsignal des stationären Detektors der Ereignisfall in einem bestimmten Raumbereich festgestellt werden und somit der zuvor beschriebene Einsatz der optischen Sensoren aktiviert bzw. getriggert werden.

Des Weiteren kann das System eine Mehrzahl von stationären Detektoren im Überwachungsbereich aufweisen, um eine Mehrzahl möglicher Leckagestellen zu überwachen und den Einsatz der optischen Sensoren damit in verschiedenen Raumbereichen zu triggern.

In weiter bevorzugter Weise kann das System dazu eingerichtet sein, im Ereignisfall aus der Mehrzahl von Detektoren einen zum ersten Raumwinkel des ersten optischen Sensors korrespondierenden stationären Detektor auszuwählen. Das System plausibilisiert die Identifikation des Zielstoffs, wenn auch der stationäre Detektor einen Zielstoff identifiziert. Alternativ oder ergänzend kann auch vorgesehen sein, dass das System dazu eingerichtet ist, stationäre Messdaten von einem externen stationären Detektor zu empfangen, um eine Identifikation des Zielstoffs zu plausibilisieren. So kann das System vorteilhaft zu einem vorhandenen Gasüberwachungssystem mit einem oder mehreren stationären Detektoren nachgerüstet werden. Stationäre Messdaten der externen stationären Detektoren können so auch von dem zuvor beschriebenen System verwendet werden. Das Ansteuern des stationären Detektors kann dabei vorzugsweise ein Empfangen von Messdaten des stationären Detektors, ein Abrufen von Messdaten des stationären Detektors, ein Aktivieren des stationären Detektors zum Erfassen von Messdaten und/oder ein Empfangen und/oder Abrufen von Messdaten von einer mit dem stationären Detektor verbundenen Auswerteeinheit sein.

Vorzugsweise ist der stationäre Detektor ausgebildet als elektrochemische Detektoren, auch als Gassensoren bezeichnet, PID-Detektoren (Photoionisationsdetektoren), FID-Detektoren (Flammenionisationsdetektoren), Wärmetönungsdetektoren, Dräger Chip-Messsysteme (CMS), auch als zur optoelektronischen Erfassung von Reaktionsprodukten von Gasen bezeichnet, direktanzeigende Dräger-Röhrchen, Mehrgasmessgeräte mit mehrere Einzeldetektoren auch unterschiedlicher Bauart, Infrarot-Sensoren, die als kleine Gasmesszellen mit breitbandiger IR-Strahlung und einem Doppeldetektor (Mess- und Referenzdetektor) ausgebildet sind, Laserspektroskop-Detektoren, Infrarot-Kameras mit Filtern, UV- und VIS-Spektrometer (z.B. Gitterspektrometer, oder Spektrometer mit optischem Spalt), LIDAR-Sensoren, photoakustische Spektroskopie-Detektoren, Nahinfrarot-Detektoren, akustische Detektoren zur Leckageortung, Massenspektrometer, Ionenmobilitätsspektrometer oder als Gaschromatographen.

Die zuvor erläuterten Ausführungsbeispiele weisen optische Sensoren und Detektoren auf, die ortsfest innerhalb der zu überwachenden Anlage oder auf dem zu überwachenden Gelände installiert und somit in einer festen räumlichen Beziehung zueinander angeordnet sind.

In einer weiteren bevorzugten Ausgestaltung des Systems kann mindestens ein Sensor als mobiler Sensor ausgebildet ist, wobei der mobile Sensor als optischer Sensor oder als Detektor ausgebildet ist.

Der mobile Sensor kann handgetragen, auf einem erdgebundenen ferngesteuerten oder bemannten Fahrzeug, oder mittels eines Fluggerätes, wie beispielsweise einer Drohne, bewegt werden. Der mobile Sensor ist bevorzugt dazu ausgebildet, während der Bewegung Messungen durchzuführen. Ferner bevorzugt ist das System eingerichtet, von dem mobilen Sensor erfasste Messdaten und/oder Messsignale einem korrespondierenden Positionswert des mobilen Sensors zuzuordnen.

Wenn der mobile Sensor selbst als optischer Sensor der zuvor beschriebenen Art ausgebildet ist, dann kann der optische als zweiter Sensor zusammen mit einem ersten stationären optischen Sensor das System bilden.

Wenn der mobile Sensor als zuvor beschriebener Detektor ausgebildet ist, dann können in einem von den mindestens zwei optischen Sensoren festgestellten Raumbereich des Ereignisses die Konzentration des Zielstoffes oder der Zielstoffe direkt durch den mindestens einen Detektor gemessen werden. Diese Vorort-Messung kann einer Plausibilisierung der vom System ermittelten Daten oder auch einer konkreten Feststellung von Konzentrationen Vorort dienen.

Das System ist vorzugsweise somit eingerichtet im Ereignisfall, wenn der erste optische Sensor in einem ersten Raumwinkel einen Zielstoff identifiziert, den mobilen Sensor in den Überwachungsbereich des ersten Sensors zu bewegen, um einen Zielstoff zu identifizieren.

Das oben aufgezeigte technische Problem wird erfindungsgemäß gelöst durch ein System zur Überwachung eines Luftraumes für ein Gelände mit mindestens einem optischen Sensor mit einem passiven Fourier-Transform-Infrarotspektrometer und mit einem Server zur Auswertung der Messdaten und zur Steuerung des mindestens einen optischen Sensors, wobei der mindestens eine optische Sensor einen einstellbaren Überwachungsbereich aufweist, wobei mindestens ein mobiler Detektor vorgesehen ist und wobei der Server eingerichtet ist, den optischen Sensor im Regelfall zu einem automatischen Scannen der Überwachungsbereiche anzusteuern, wobei der Server den Messdaten jeweils einen Raumwinkel anhand der Positionsdaten des optischen Sensors zuordnet, aus den Messdaten des optischen Sensors für jeden Raumwinkel die spektrale Intensitätsverteilung der empfangenen IR-Strahlung abzuleiten und mittels Korrelation der Intensitätsverteilung mit bekannten Gasspektren mindestens einen Zielstoff zu identifizieren und im Ereignisfall, wenn der optische Sensor in einem Raumwinkel einen Zielstoff identifiziert, mit dem mindestens einen mobilen Detektor die Konzentration des Zielstoffes entlang des vom optischen Sensor identifizierten Raumwinkels ortsabhängig zu erfassen.

Somit ist das System und vorzugsweise der Server dazu eingerichtet, den mobilen Detektor zum Lokalisieren des Zielstoffs in dem Überwachungsbereich des ersten Sensors zu bewegen. Vorzugsweise ist das System eingerichtet, den mobilen Detektor entlang einer vorbestimmten Bahn durch den Überwachungsbereich zu bewegen. Vorzugsweise kann die vorbestimmte Bahn ein Raster sein. Vorzugsweise ist das System eingerichtet, den mobilen Detektor entlang einer Bewegungsbahn zu bewegen, die zu einer optischen Achse des ersten optischen Sensors im ersten Raumwinkel korrespondiert. Das System lokalisiert den Zielstoff vorzugsweise durch Ermitteln derjenigen Position des mobilen Detektors im Überwachungsbereich des ersten Sensors, für die die Konzentration des Zielstoffs maximal ist.

Das zuvor beschriebene System zur Überwachung eines Luftraumes für ein Gelände nutzt optische Sensoren vorzugsweise mit einem FTIR-Spektrometer zur Detektion von Zielstoffen, mit einer Infrarotoptik zur Abbildung eines Teilausschnitts des zu überwachenden Luftraums des Geländes auf das FTIR-Spektrometer, mit einer Videokamera und mit einer Positioniereinheit zur Ausrichtung der aus dem FTIR-Spektrometer, der Infrarot-Optik und der Kamera gebildeten Sensoreinheit, wobei die Infrarot-Optik und die Kamera einen gleichen Raumwinkel erfassen, insbesondere wobei die optischen Achsen der Infrarot-Optik und der Kamera parallel zueinander ausgerichtet sind.

Damit wird ein optischer Sensor angegeben, der als Ganzes in verschiedene Raumwinkel verstellt werden kann und somit nahezu keine Einschränkungen der Beobachtungsrichtungen aufweist. Somit wird der optische Sensor kleiner und lässt sich einfacher installieren, als es bei aus dem Stand der Technik bekannten optischen Sensoren mit FTIR-Spektrometer der Fall ist.

Insbesondere ist die Infrarot-Optik als Cassegrain-Teleskop mit einem Parabolspiegel und mit einem Sekundärspiegel ausgebildet. Die Ausbildung als Cassegrain-Teleskop vereinfacht die Abbildung des Teilausschnittes des zu überwachenden Luftraums und führt wegen der Anwendung des Parabolspiegels zu einer erhöhten Intensität der aufgenommenen Strahlung und somit auch des FTIR-Signals.

In vorteilhafter Weise ist die Kamera seitlich zur Infrarot-Optik angeordnet. Daher kann die Kamera, die ebenfalls den Teilausschnitt des Geländes abbilden soll, gegebenenfalls ein Teleobjektiv aufweisen und ein vergrößertes Bild erzeugen. Die Kamera beeinträchtigt dann nicht den optischen Weg der Infrarot-Optik, weist aber eine Parallaxe auf, die für eine genaue Überlagerung der Messergebnisse mit dem Kamerabild ausgeglichen werden muss.

Alternativ kann die Kamera auch in der optischen Achse der Infrarot-Optik angeordnet sein, insbesondere am oder vor dem Sekundärspiegel. Somit kann die Kamera ohne Parallaxenverschiebung das Videobild aufnehmen, ohne den optischen Weg der Infrarot-Optik zu beeinflussen.

Eine weitere Alternative besteht darin, dass die Kamera als Kamerasystem mit einer seitlich zur Infrarot-Optik angeordneten Kamera und mit einer in der optischen Achse der Infrarot-Optik angeordneten Kamera ausgebildet ist. Damit werden die Vorteile der beiden Kameras miteinander kombiniert, die seitliche Kamera kann ein hoch aufgelöstes Bild erzeugen und die in der optischen Achse angeordnete Kamera dient der Parallaxen-freien Bilderzeugung.

Vorzugsweise weist die Positioniereinheit eine Kommunikationseinrichtung auf und ist eingerichtet, auf Basis von durch die Kommunikationseinrichtung empfangenen Steuerbefehlen die Sensoreinheit auszurichten.

Die Positioniereinheit ist somit eine fernsteuerbare Positioniereinheit ist vorzugsweise zum drahtlosen Empfangen von Steuersignalen ausgebildet. In einer bevorzugten Ausgestaltung weist die fernsteuerbare Positioniereinheit eine Antenneneinheit zum Empfangen von Steuersignalen und zum Senden von Messdaten und/oder Messsignalen auf. So kann eine physische Verbindung der optischen Sensoren vermieden werden. Ein Installieren des Systems ist dann auch mit großem Abstand der optischen Sensoren zueinander schnell und unkompliziert möglich. Auch optische Sensoren mit fernsteuerbarer Positioniereinheit können eine physische bzw. leitungsgebundene Stromversorgung aufweisen.

Bevorzugt weist der optische Sensor eine Kommunikationseinrichtung zum Empfangen und/oder Senden von Signalen auf. Signale können analoge und/oder digitale Signale sein. Die Kommunikationseinrichtung kann leitungsgebunden und/oder drahtlos ausgebildet sein.

Vorzugsweist ist die Kommunikationseinrichtung zum Empfangen und/oder Senden von Signalen via WLAN oder Mobilfunk, insbesondere GPRS, UMTS, LTE, LTE-Advanced, 5G ausgebildet. Bevorzugt ist die Positioniereinheit dazu eingerichtet, unter Verwendung der empfangenen Signale die Infrarot-Optik zum Scannen eines Überlappungsbereichs mit einem Überwachungsbereich eines weiteren Sensors automatisiert zu bewegen.

Vorzugsweise ist der optische Sensor dazu angepasst, Zielstoffdetektionssignale eines weiteren Sensors zu Empfangen und in Antwort auf ein Zielstoffdetektionssignal automatisiert einen Überlappungsbereich mit einem Überwachungsbereich des weiteren Sensors zum Detektieren eines Zielstoffs zu scannen. Das Zielstoffdetektionssignal umfasst zumindest die Information, dass der weitere Sensor einen Zielstoff identifiziert hat. Ferner umfasst das Zielstoffdetektionssignal vorzugsweise den Raumwinkel, für den der weitere Sensor den Zielstoff identifiziert hat. Vorzugsweise ist der optische Sensor, in Antwort auf das Identifizieren eines Zielstoffs in dessen Überwachungsbereich, zum Senden eines Zielstoffdetektionssignals eingerichtet. Ein Empfangen eines Zielstoffdetektionssignals kann alternativ oder ergänzend zu einem unmittelbaren Empfangen von einem weiteren optischen Sensor auch ein mittelbares Empfangen über eine Einheit, beispielsweise einen Server, sein.

Bei einem Verfahren zur Überwachung eines Luftraumes für ein ausgedehntes Gelände wird mit mindestens zwei optischen Sensoren mit einem passiven Fourier-Transform-Infrarotspektrometer das Gelände zumindest abschnittsweise überwacht, werden mit jedem optischen Sensor jeweils innerhalb eines Überwachungsbereiches einstellbare Raumwinkelbereiche erfasst, bei dem sich der Überwachungsbereich eines optischen Sensors mit dem Überwachungsbereich mindestens eines weiteren optischen Sensors zumindest abschnittsweise überlappt, werden die optischen Sensoren im Regelfall zu einem automatischen Scannen der Überwachungsbereiche angesteuert, wird aus den Messdaten der optischen Sensoren für jeden Raumwinkel die spektrale Intensitätsverteilung der empfangenen IR-Strahlung abgeleitet und eine Korrelation der Intensitätsverteilung mit bekannten Gasspektren durchgeführt, wird im Ereignisfall, wenn von einem ersten optischen Sensor in einem ersten Raumwinkel ein Infrarotsignal eines Zielstoffes identifiziert wird, mindestens ein weiterer optischer Sensor angesteuert, den Überlappungsbereich mit dem Überwachungsbereich des ersten optischen Sensors zu scannen, wird aus den Messdaten des mindestens einen weiteren optischen Sensors mindestens ein weiterer Raumwinkel mit einem Infrarotsignal des Zielstoffes identifiziert und werden aus den Raumwinkelinformationen des ersten Raumwinkels und des mindestens einen weiteren Raumwinkels die Koordinaten des Überlappungsbereiches mit erhöhter Konzentration des Zielstoffes ermittelt. Bei dem Verfahren weist der Überwachungsbereich jedes optischen Sensors durch die Topographie und/oder die Bebauung des Geländes aufgrund von Abschattungen abhängig vom Raumwinkel unterschiedliche Messradien auf und wird der Überwachungsbereich jedes optischen Sensors durch den Raumwinkelbereich und die zugehörigen Messradien bestimmt und festgelegt und werden Messsignale des mindestens einen weiteren optischen Sensors in Raumrichtungen mit zu geringem Messradius nicht in die Auswertung einbezogen.

Bei der Durchführung des Verfahrens kann insbesondere mindestens ein oben beschriebener optischer Sensor eingesetzt werden.

In bevorzugter Weise werden die Messsignale des mindestens einen weiteren optischen Sensors, die nicht in die Auswertung einbezogen worden sind, durch mathematische Interpolation von angrenzenden Messsignalen ersetzt.

Des Weiteren können mindestens drei optische Sensoren eingesetzt werden, wobei von dem ersten Sensor in dem ersten Raumwinkel ein Infrarotsignal eines Zielstoffes identifiziert wird und wobei im Ereignisfall zumindest der weitere optische Sensor für eine Ansteuerung ausgewählt wird, dessen Überwachungsbereich eine maximale Überlappung mit dem Überwachungsbereich des ersten optischen Sensors aufweist.

Dabei ist es vorteilhaft, wenn ein ausgewählter optischer Sensor nicht eingesetzt wird, wenn die Position des ausgewählten optischen Sensors in Richtung des Raumwinkelbereichs des ersten optischen Sensors mit nachgewiesenem Zielstoff liegt. Denn in diesem Fall besteht kein für eine Triangulation ausreichender Winkel zwischen dem Raumwinkel des detektierten Zielstoffes und dem notwendigen Raumwinkel des weiteren optischen Sensors. In einem solchen Fall kann dann der nächstbeste optische Sensor ausgewählt werden, der eine möglichst große Überlappung mit dem Überwachungsbereich des ersten optischen Sensors aufweist.

Weiterhin ist es bevorzugt, dass die Säulendichte als mathematisches Produkt aus Konzentration des Gases (gemessen in ppm) und der Länge der Gaswolke (gemessen in m) berechnet wird. Somit lässt sich einem detektierten Zielstoff eine Konzentration beimessen, nachdem die Länge der Wolke mit dem zuvor beschriebenen Verfahren bestimmt worden ist.

In weiter bevorzugter Weise werden im Ereignisfall die optischen Sensoren für Messungen mit einer erhöhten Ortsabtastung angesteuert und/oder die Koordinaten des Überlappungsbereiches der höchsten Säulendichten verschiedener optischer Sensoren ermittelt und/oder die Koordinaten mit einer Kartendarstellung verknüpft und eine zweidimensionale Darstellung des Ereignisses erstellt und/oder die Koordinaten mit den Bildern einer Videokamera verknüpft und eine visuelle Darstellung des Ereignisses erstellt und/oder mit mindestens einer aktiven Infrarotstrahlungsquelle das Infrarotlicht von einem optischen Sensor aufgenommen.

Bevorzugt wird ein Verfahren, bei dem stationäre Messdaten, vorzugsweise stationäre Messdaten eines externen stationären Sensors, empfangen werden, und bei dem im Ereignisfall die Identifikation des Zielstoffs unter Verwendung der stationären Messdaten plausibilisiert wird.

Für die zuvor beschriebenen Systeme und das beschriebene Verfahren ist also erkannt worden, dass durch das Ansteuern eines weiteren optischen Sensors eine Genauigkeit der Erfassung von Zielstoffen erheblich gesteigert werden kann. Ferner ist erkannt worden, dass bei einem Gelände mit einer Topographie, die den freien Blick der optischen Sensoren beeinträchtigt, die Lokalisation des Störereignisses schnell und zuverlässig erfolgen kann, wenn die Topographie des Geländes bei der Installation des Systems für die Bestimmung der Überwachungsbereiche berücksichtigt wird. Denn nicht nur die Raumrichtungen, sondern auch die zugehörigen Messradien, die durch das Gelände, durch Gebäude und/oder durch andere technische Anlagen begrenzt werden, werden zur Bestimmung der Überwachungsbereiche herangezogen. Denn die optischen Sensoren können nur so weit messen, wie freie Sicht besteht. Die Lokalisierung des Störereignisses erfolgt dann nur mit den Messdaten, deren räumliche Erstreckungen sich auch tatsächlich überlappen.

Nachfolgend werden das beschriebene System, der optische Sensor und das beschriebene Verfahren im Detail erläutert.

Die beschriebenen Systeme und Verfahren dienen zur echtzeitnahen Identifikation und Lokalisierung von Gasleckagen und zur kartenbasierten Lagebewertung für Einsatzkräfte im Ereignisfall, sowie zur kontinuierlichen Emissions- sowie Immissionsmessung gasförmiger Gefahrstoffe innerhalb oder entlang des Perimeters eines Überwachungsbereiches.

Die Systeme, die auch Luftraumüberwachungssystem genannt werden können, basieren auf der Vernetzung von mindestens zwei, prinzipiell aber von einer beliebigen Anzahl an passiven, frei positionierbaren FTIR-Fernerkundungsspektrometern als optische Sensoren zu einem bi- bzw. multiperspektivischen Gesamtsystem.

Optional kann die passive Infrarotspektroskopie mit einzelnen aktiven Infrarotmessstrecken kombiniert werden.

Durch die passive Messtechnik wird die flächendeckende Abtastung eines großen Überwachungsbereiches bzw. des Luftraums ermöglicht. Das Hinzuziehen einzelner aktiver Messstrecken ermöglicht die hochgenaue Hintergrundkonzentrationsbestimmung einer Vielzahl von Gasen (Emissions- und Immissionsmessung) sowie eine erweiterte Zielstoffbibliothek und niedrigere Nachweisgrenzen entlang der vordefinierten aktiven Messstrecken. Aktiver und passiver Modus können unabhängig voneinander und mit demselben optischen Sensor betrieben werden.

Im Folgenden wird der Aufbau des Systems erläutert.

An ausgewählten Orten, vorzugsweise mit gutem Überblick über das zu überwachende Gelände, z.B. auf Dächern, an Kaminen oder an Masten, werden optische Sensoren unter freiem Himmel fest, aber hinsichtlich ihrer Ausrichtung frei positionierbar für die Dauerüberwachung installiert. Ein optischer Sensor ermöglicht im passiven Anwendungsfall einen Messradius von bis zu ca. 1 - 4 km, je nach gewünschter örtlicher Auflösung und freie Sicht vorausgesetzt. Die Scangeschwindigkeiten und die erreichbaren Auflösungen bedingen einander, eine typische Scangeschwindigkeit eines 360°-Scans liegt im Bereich von 1 bis 10 Minuten. Daher werden im Anwendungsfall Pfade festgelegt, die beim Scannen von einem optischen Sensor durchlaufen werden. Kritische Anlagenteile werden dabei intensiver, also mit größeren Raumwinkelbereichen erfasst, als es beispielsweise für Verwaltungsgebäude erforderlich ist, die gegebenenfalls sogar ganz oder teilweise aus dem Pfad herausgenommen werden.

Um die verfahrensmäßige kombinierte Auswertung von den mindestens zwei optischen Sensoren zu ermöglichen, überlappen sich die Überwachungsbereiche zumindest eines Teils der optischen Sensoren. Optional werden die aktiven Infrarotstrahlungsquellen entlang ausgewählter Messstrecken in Entfernungen von bis zu einigen hundert Metern fest installiert und auf den ihnen zugeordneten optischen Sensor ausgerichtet.

Die optischen Sensoren arbeiten im Routinefall dezentral und autonom, die Messdaten laufen jedoch bevorzugt in einem Server als Zentralsystem zusammen, was die kombinierte Auswertung und die Steuerung als Gesamtsystem ermöglicht.

Die optischen Sensoren stellen einzelne Messstellen des Gas-Ferndetektionssystems dar und verwenden FTIR-Spektrometer zur Ferndetektion von Gasen in Kombination mit einer Infrarotoptik zur Reduktion des Gesichtsfeldes des Spektrometers, einer Positioniereinheit zur Ausrichtung des Spektrometers sowie einer Videokamera und einer vorzugsweise im optischen Sensor oder im externen Server in einer der zuvor erläuterten Ausführungsformen gespeicherten Auswertungs- und Steuerungssoftware. Die Positioniereinheit ermöglicht eine freie Ausrichtung in 360° Azimut und mindestens +/- 60° Elevation.

Das FTIR-Spektrometer ist vorzugsweise nicht von der Positioniereinheit getriggert, sondern liefert einen kontinuierlichen Strom von Messdaten. Es kann aber auch vorgesehen sein, dass der optische Sensor nur im Ereignisfall kontinuierlich Messdaten bereitstellt und während eines Scannens für regelmäßig zueinander beabstandete Raumwinkel Messdaten bereitstellt. Beispielsweise könnte in 2° Schritten Messdaten bereitgestellt werden, wodurch gegenüber einer kontinuierlichen Messdatenerfassung Auswertekapazitäten eingespart werden können. Die Positioniereinheit bewegt somit das FTIR-Spektrometer zusammen mit der Infrarot-Optik und der Kamera als eine Sensoreinheit. Durch die Anpassung der Winkelgeschwindigkeit der Positioniereinheit wird die Ortsabtastung der Messung gesteuert. Durch die Zuordnung der Positionszeitstempel der Positioniereinheit werden die Messdaten einem Raumwinkel zugeordnet. Zudem kann ein Messzeitstempel zugeordnet werden, um eine zeitliche Entwicklung der Zielstoff-Wolke zu erfassen.

Eine Videokamera, die als Teil des optischen Sensors gemeinsam mit dem FTIR-Spektrometer ausgerichtet ist, liefert entweder kontinuierlich oder bei vordefinierten Positionen ein Videobild in Blickrichtung des FTIR-Spektrometers. Benachbarte Videobilder können mithilfe eines Stitching-Algorithmus zu einem zusammenhängenden Videobild beliebiger Größe und mit beliebigem Anzeigeformat bis zu einer 360° Rundumsicht zusammengesetzt werden.

Durch die Überlagerung von chemischen bzw. spektroskopischen Informationen, also der Messergebnisse mit dem Videobild, wird eine zweidimensional ortsaufgelöste Gasverteilung aus den Messdaten visualisiert. Eine bilineare Interpolation der Messergebnisse in X- und Y-Richtung, also über das zweidimensionale Bild, welches von dem optischen Sensor abgetastet wurde, und die zusätzliche oder alternative Überlagerung mit einem Videobild, liefert eine intuitiv erfassbare zweidimensionale Ansicht der identifizierten Gaswolke.

Unabhängig von den Einstellungen der gemessenen Winkelbereiche ist die Darstellung der Ergebnisbilder für frei wählbare Teilbereiche innerhalb des gemessenen Winkelbereichs in frei wählbaren Anzeigeverhältnissen möglich.

Im zeitlichen Verlauf generiert der optische Sensor einen Stream aus Ergebnisbildern sowie einen Stream aus zeit- und raumwinkelgestempelten Messergebnissen.

Die Gas-Fernerkundung mittels Infrarotspektroskopie misst langwellige Infrarotstrahlung und wertet das gemessene Infrarotspektrum auf das Vorhandensein bekannter spektraler Signaturen von Zielstoffen aus. Auf diese Weise lassen sich gasförmige Zielstoffe aus großen Entfernungen von bis zu mehreren Kilometern identifizieren und auch quantifizieren.

Das FTIR-Spektrometer empfängt Infrarotstrahlung, die mittels einer Infrarot-Optik, beispielsweise ein Cassegrain-Teleskop oder eine Linsenoptik, empfangen, in das FTIR-Spektrometer eingekoppelt, durch ein Michelson-Interferometer geleitet und auf die Detektorebene fokussiert wird. Mittels des Interferometers wird ein Interferogramm gemessen, welches durch Fourier-Transformation in ein Infrarotspektrum umgerechnet wird. Der Bewegungsbereich des Scanspiegels innerhalb des Interferometers liegt typischerweise im Bereich von 0,75 bis 10 mm.

Ein geeignetes FTIR-Spektrometer verwendet beispielsweise Cryo-gekühlte Mercury-Cadmium-Tellurid (MCT) Einzeldetektoren in Kombination mit einer radiometrischen Kalibriereinheit. Auf Grundlage einer radiometrischen Kalibrierung wird das gemessene Infrarotspektrum in ein kalibriertes Strahlungstemperaturspektrum umgerechnet, welches Eingang in den spektralen Auswertealgorithmus findet.

Zur spektralen Auswertung werden die spektralen Signaturen des Zielstoffes und von atmosphärischen Störstoffen, wie z.B. Wasser, CO₂ und anderen Gasen, sowie mathematische Funktionen zur Modellierung des Hintergrundes berechnet. Im Ergebnis an diese Anpassungsrechnung werden die berechnete Signalhöhe und der Korrelationskoeffizient der berechneten Zielstoffsignatur mit stoffspezifischen Grenzwerten verglichen. Bei Überschreitung von vorgegebenen Grenzwerten gilt der Zielstoff als identifiziert, was automatisch erfolgt und direkt zu weiteren Aktionen des Systems führen kann.

Das Prinzip der passiven IR-Fernerkundung arbeitet unabhängig von künstlichen Strahlungsquellen und funktioniert sowohl tagsüber als auch nachts, unabhängig von Ort, Messrichtung und der Jahreszeit, indem die von der Umgebung emittierte Wärmestrahlung in der Peilrichtung des Spektrometers analysiert wird. Die notwendige Bedingung zur Identifikation eines Gases ist das Vorhandensein eines zumindest geringen Temperaturunterschiedes zwischen der Strahlungstemperatur des zu messenden Gases, also des Zielstoffes, und der Strahlungstemperatur des Hintergrundes in Blick- bzw. Peilrichtung. Ist das Gas wärmer als der Hintergrund in Messrichtung, erscheint die spektrale Signatur in Emission, ist es kälter als der Hintergrund, in Absorption.

Die passive Infrarot-Fernerkundung verwendet den langwelligen Spektralbereich infraroter Strahlung, beispielsweise im Wellenzahl-Bereich von ca. 700- 1.400 cm⁻¹. Innerhalb dieses atmosphärischen Fensters sind Messungen auf große Entfernungen von bis zu mehreren Kilometern möglich, ohne dass die Atmosphäre eine zu starke Abschwächung des Signals bewirkt. Als begrenzende Faktoren für die maximale Messreichweite sind allgemein zu berücksichtigen: das Gesichtsfeld des Detektors durch vorhandene Hindernisse, die Größe der zu messenden Wolke und die Abschwächung des Signals entlang der optischen Messstrecke durch die Atmosphäre, wobei eine direkte Sichtlinie stets Voraussetzung ist. In typischen Anwendungen werden Messungen mit einer Messreichweite von bis zu 1 - 5 km vorgenommen.

Für das Feststellen eines Störfalles ist es zunächst erforderlich, einen Zielstoff, also ein nicht gewünschtes Gas zu identifizieren. Dazu wird bei ausreichendem Signal-zu-Rauschen-Abstand des analysierten Messsignals am Ausgang des Interferometers ein entsprechendes Ausgangssignal erzeugt. Die Identifizierung eines Zielstoffes in einem erfassten Raumwinkel führt also zu einem Auslösen eines Ereignisfalls.

Zudem kann die Quantifizierung des Gases bzw. Zielstoffes in einer passiven Einzelmessung in Form der Bestimmung einer Säulendichte (Einheit ppm•m) erfolgen, also des Produkts der Dichte der Wolke und der Wolkenlänge in Messrichtung. Die Säulendichte entspricht somit der Dichte integriert entlang des Messweges.

Zur spektralen Quantifizierung der Säulendichte ist der Ansatz über das Lambert-Beer'sche Gesetz unter Annahme einer Gastemperatur und der Berücksichtigung des Absorptionsquerschnittes des Zielstoffes möglich. Ein alternativer Ansatz ist die Quantifizierung mittels eines nichtlinearen Fitting-Algorithmus, bei dem zusätzlich auch die Gastemperatur als Modellparameter berechnet wird.

Die aktive Messung verwendet in Abgrenzung zur passiven Messung eine oder mehrere fernaufgestellte aktive Infrarotstrahlungsquelle(n), um den Rauschabstand der Einzelmessung zu vergrößern. Dadurch wird einerseits die Messung geringerer Zielstoffkonzentrationen ermöglicht und andererseits wird der messbare Spektralbereich der Messung erweitert, wodurch eine größere Anzahl der zu messenden Stoffe ermöglicht wird. Die aktive Messung ist in ihrer Blickrichtung entlang der Messstrecke durch das Vorhandensein einer fernaufgestellten Strahlungsquelle limitiert und nicht zu jedem Zeitpunkt frei positionierbar.

Die aktiven Infrarotstrahlungsquellen sind vorzugsweise breitbandige Emitter, deren Strahlung durch eine Infrarotoptik gerichtet wird und die auf die optischen Sensoren ausgerichtet sind. Es können auch schmalbandige Emitter eingesetzt werden.

Der optische Sensor wird für die aktive Messung auf eine gerichtete Infrarotstrahlungsquelle entlang der Messstrecke ausgerichtet, die in einer Entfernung von typischerweise einigen zehn bis einigen hundert Metern installiert ist. Der optische Sensor empfängt die unmodulierte, breitbandige Infrarotstrahlung der Quelle und misst ein Infrarotspektrum. Gase, die sich zum Zeitpunkt der Messung entlang der optischen Messstrecke zwischen der Strahlungsquelle und dem optischen Sensor befanden, bewirken in Abhängigkeit ihres stoffspezifischen IR-Absorptionsquerschnittes eine Absorption der infraroten Strahlung der Quelle, welche maßgeblich von der Konzentration des Zielstoffes sowie der Länge der Wolke entlang der Messstrecke abhängt. Der spektrale thermische Hintergrund wird ermittelt und vom gemessenen Spektrum abgezogen.

Durch das Hinzuziehen der aktiven Strahlungsquelle wird der messbare Spektralbereich in Richtung kurzwelligen, energiereicheren Lichtes erweitert. Der typische Messbereich liegt im Wellenzahl-Bereich von ca. 700 - ca. 4.500 cm⁻¹.

Die Auswertung der gemessenen aktiven Infrarotspektren erfolgt mittels eines spektralen Auswertealgorithmus. Eine Kalibration des Systems mittels definierter Gaskonzentrationsstufen ist zur quantitativen Auswertung nicht erforderlich.

Das bi- bzw. multiperspektivische System, das auch als Überwachungssystem bezeichnet werden kann, wird durch die Kombination von mindestens zwei, vorzugsweise von mehreren passiven FTIR-Spektrometern und optional von breitbandig emittierenden gerichteten Infrarotstrahlungsquellen sowie einem zentralen Server realisiert. Damit können Gaswolken identifiziert und lokalisiert sowie Gaskonzentrationen quantifiziert werden.

Das System dient zur Lokalisierung von Gefahrenbereichen im Falle von toxischen oder anderweitig gefährlichen Gasen und zur Umweltüberwachung von Emissions- und Immissionswerten innerhalb und/oder entlang des Perimeters eines zu überwachenden Geländes.

Die Verwendung passiver FTIR-Spektrometer zur Ferndetektion von Gasen ermöglicht somit den Aufbau eines abstandsfähigen Überwachungssystems. Damit kann einerseits ein hoher Abdeckungsgrad des Geländes erreicht werden. Andererseits ist die Anpassung des Überwachungsbereiches der optischen Sensoren in Echtzeit und situationsbezogen möglich.

Das Gas-Ferndetektionssystem überwacht autonom Bereiche des Geländes in einem Radius von beispielsweise bis zu 4 km je optischen Sensor. Die Anzahl der kombinierten optischen Sensoren zum Gesamtsystem ist beliebig, mindestens sind jedoch zwei passiv arbeitende optische Sensoren vorhanden.

Für jeden optischen Sensor wird der Überwachungsbereich anhand der Topographie des Geländes eingerichtet. Dabei wird einerseits der zu überwachende Raumwinkelbereich in Breite und Höhe festgelegt und andererseits werden für alle Raumrichtungen die Messradien bestimmt. Denn durch topographische Hindernisse wie Geländeerhebungen, Gebäude und/oder Anlagen und deren Teile wird das Sichtfeld jedes optischen Sensors begrenzt.

Zudem wird für jeden optischen Sensor festgelegt, welcher Pfad innerhalb des Überwachungsbereiches während des Regelfalls der Überwachung gescannt werden soll, also welche Azimut- und Elevationswinkel durchlaufen werden sollen.

Die Verwendung des FTIR-spektroskopischen Messverfahrens erlaubt die Detektion und Identifikation einer sehr großen Anzahl von Chemikalien, deren charakteristische Spektren in einer Stoffbibliothek auf dem Server abgelegt sind. Im Fall der Präsenz eines Zielstoffes der Stoffbibliothek identifiziert das System Ort und gegebenenfalls auch die zeitliche Entwicklung der Ausbreitung und die Ausbreitungsrichtung der Zielstoff-Gaswolke im Raum. Dazu werden mindestens zwei, ggf. auch alle im System integrierten optischen Sensoren automatisch in eine situationsbezogene Lageeinschätzung einbezogen.

Durch die messtechnische Erfassung elektromagnetischer Strahlung über einen sehr breiten Spektralbereich mit hoher spektraler Selektivität können in derselben Messung viele verschiedene Zielstoffe eindeutig identifiziert werden. Die Zielstoffdatenbank des Systems kann bis zu mehrere hundert Chemikalien umfassen. Vorzugsweise sind als typische Chemikalien Ammoniak, Methanol, Methan, Chloroform und Ethylen zu nennen.

So eignet sich das System für viele unterschiedliche Szenarien und kann auf veränderliche Produktionsprozesse, Lagerzyklen von Chemikalienlagern oder veränderliche Störeinflüsse angepasst werden. Darüber hinaus bilden die gemessenen Daten der Infrarotspektren einen physikalischen Ist-Zustand ab, der als Stoffnachweis und Stoffnichtnachweis dienlich ist.

Ein Bewertungsalgorithmus auf dem Server, insbesondere der Auswertungs- und Steuerungseinheit, verarbeitet die Stoffinformationen und Spektraldaten der optischen Sensoren und der gegebenenfalls ermittelten Säulendichten und steuert den Messablauf. Die beschriebene Steuerung und Koordination der optischen Sensoren minimiert die Überwachungszeit, ereignisbezogen werden potenzielle Gasdetektionen aus allen möglichen Blickrichtungen validiert, zudem werden potenzielle Ereignisse von Störgrößen oder Einzeldetektionen unterschieden und bei Präsenz einer potenziell gefährlichen Gaswolke wird eine spezifische und eindeutige Alarmierung ausgeben.

Gaswolken können im Alarm- bzw. Ereignisfall in einer 2D Kartendarstellung und/oder einer 2D-Visualisierung als Überlagerung der Gaswolke mit einem Videobild aus der Blickrichtung des optischen Sensors abgebildet werden.

Das ausgedehnte Gelände wird in vordefinierte Überwachungsbereiche unterteilt, die im Routinefall automatisiert von den jeweiligen Einzelsystemen entlang frei programmierbarer Messpfade wiederkehrend und unabhängig von den übrigen Systemen abgescannt werden.

Im Ereignisfall, d.h. nach Identifikation eines Zielstoffes durch mindestens einen optischen Sensor, werden ausgewählte, benachbarte optische Sensoren automatisiert hinzugezogen, um das Ereignis zu verifizieren sowie um die Gaswolke zu lokalisieren und zu quantifizieren. Somit wird eine echtzeitnahe kartenbasierte Lagebewertung des Ereignisses generiert und z.B. für die Einsatzkräfte zur Verfügung gestellt.

Die Auswahl derjenigen optischen Sensoren, die im Ereignisfall hinzugezogen werden, erfolgt auf Grundlage a priori ermittelter gemeinsamer Überwachungsbereiche bzw. Sichtfeldbereiche, die sich aus der Topographie des überwachten Geländes und der Position der optischen Sensoren ergeben sowie aus der Blickrichtung, in welcher das Ereignis festgestellt wurde. Der Einfluss der Topographie durch Abschattungen der Sichtbereiche der optischen Sensoren durch Geländeerhebungen, Gebäude und/oder Anlagen wird berücksichtigt, so dass in Sichtrichtung hinter einem topographischen Hindernis keine Messwerte der entsprechenden optischen Sensoren erlangt werden können. Diese topographische Information ist bei der während der Installation der optischen Sensoren erfolgten Bestimmung des jeweiligen Überwachungsbereiches berücksichtigt worden.

Die jeweiligen Überwachungsbereiche und die gemeinsamen sich überlappenden Überwachungsbereiche der installierten optischen Sensoren werden somit zum Installationszeitpunkt berechnet und abgelegt, so dass diese im Ereignisfall nur noch abgerufen und mit der Blickrichtung des Ereignisses abgeglichen werden müssen, eine Neuberechnung sich überlagernder Überwachungsbereiche bzw. Sichtfeldbereiche ist im Normalfall nicht erforderlich.

Auf Basis der bekannten Überwachungsbereiche bzw. Sichtfeldbereiche werden die weiteren optischen Sensoren automatisiert zur Zielfindung gesteuert. Dazu wird der mindestens eine weitere optische Sensor so angesteuert, dass er den Überwachungsbereich des den Ereignisfall auslösenden optischen Sensors scannt.

Die gemeinsame Identifikation eines Zielstoffs aus mehr als einer Messposition ermöglicht somit die Verifikation eines potenziellen Ereignisses und die Lokalisation der identifizierten Zielstoff-Gaswolke im Raum durch Triangulation. Des Weiteren kann die Berechnung einer räumlichen Konzentrationsverteilung der Gaswolke erfolgen. Eine nachgeschaltete Anwendung von Bewertungskriterien zur automatisierten Interpretation der Messergebnisse basierend auf einer Güteeinschätzung der Messwerte sowie räumlicher und zeitlicher Parameter erlaubt eine belastbare Lageeinschätzung.

Im Ergebnis werden die Messdaten kartenbasiert als farblich gekennzeichnete Lagebewertung dargestellt. Zusätzlich kann jederzeit die dem Videobild überlagerte zweidimensionale Darstellung der Messergebnisse ausgewählter optischer Sensoren in Form eines zeitlichen Videostreams hinzugezogen werden.

Sobald ein optischer Sensor bzw. das System die Identifikation eines Zielstoffs aus der Zielstoffbibliothek signalisiert, verlässt das System seinen Routinebetrieb und wechselt in die Zielfindung und anschließende Lagebewertung eines potenziellen Ereignisfalls.

Bei Identifikation eines Zielstoffes aus dem Sichtbereich eines optischen Sensors wird mindestens ein weiterer optischer Sensor bzw. werden die übrigen optischen Sensoren zur Gewinnung von weiteren Informationen koordiniert. Dazu werden die vordefinierten Messpfade bis zur Wiederaufnahme des Normalbetriebs im Routinebetrieb verlassen.

Die Analyse der örtlichen Verteilungsfunktion des Messsignals liefert Winkelbereiche, für die eine Präsenz einer Gaswolke wahrscheinlicher ist. Diese Winkelbereiche werden vorzugsweise in höherer örtlicher Auflösung abgetastet.

Winkelbereiche können klassifiziert werden in Bereiche, in denen (a) ein Zielstoff identifiziert wird, (b) Winkelbereiche, in denen messtechnisch keine Identifikation möglich ist, und (c) Winkelbereiche, die eine messtechnische Identifikation eines Zielstoffes zulassen und für die nach der Bewertung des Identifikationsalgorithmus in Bezug auf das erzeugte Messsignal und gegebenenfalls die minimale Säulendichte des Zielstoffs der Zielstoff nicht nachgewiesen wird. Die Klassifizierung der Überwachungsbereiche ist stoffspezifisch.

Die Darstellung eines Überwachungsbereiches kann in einer Karte erfolgen. Bereiche in einem Sichtfeld eines optischen Sensors werden in der Karte dargestellt. Im Fall einer Identifikation eines Zielstoffes aus der verwendeten Zielstoffbibliothek wird der Sichtbereich in der Karte markiert. Im Fall einer Lokalisation einer Gaswolke durch die gemeinsame Identifikation aus mehr als einer Messposition wird der Bereich, in dem die Gaswolke lokalisiert wird, in der Karte markiert.

Durch die Analyse der Verteilungsfunktion der Messsignale, gegebenenfalls der berechneten Säulendichten des Zielstoffs sowie der Analyseparameter für die Zielstoffidentifikation und unter Berücksichtigung der Nachweisgrenzen für jeden Messpunkt können die jeweiligen Raumwinkel ermittelt werden, in denen jeder optische Sensor, der einen Zielstoff identifiziert, die höchste Säulendichte des Zielstoffs ermittelt. Dieser Messwert dient zur Lokalisation des potenziellen Punktes der Gaswolke mit der höchsten Zielstoffkonzentration.

Bei gemeinsamer Stoffidentifikation von mehr als einem optischen Sensor kann aus allen Raumwinkeln, für die eine maximale Säulendichte ermittelt wurde, ein potenzieller Schwerpunkt für die Gaswolke ermittelt werden. Dieser messtechnisch ermittelte Punkt kann weiterhin auf Grundlage der Messparameter klassifiziert werden:
(a) Unter der Bedingung eines freien Sichtfeldes auf die Gaswolke aus mindestens zwei Messpositionen und unter der Bedingung, dass die ermittelte Säulendichte für den potenziellen Schwerpunkt der Gaswolke für jeden Sichtbereich eines optischen Sensors größer als die Nachweisgrenze der übrigen Messpunkte des Überwachungsbereichs ist, handelt es sich bei dem erfassten Punkt um den Ort der Gaswolke, der die höchste Konzentration aufweist.
(b) Sofern es Bereiche innerhalb der Gaswolke gibt, die entweder auf Grund von Abschattungen oder aufgrund einer geringen Nachweisgrenze für den jeweiligen betrachteten Raumwinkel messtechnisch nicht aus mindestens zwei Messpositionen erfassbar sind, wird ermittelt, ob sich für die jeweiligen Raumwinkel mit höchster ermittelter Säulendichte ein gemeinsamer Schnittpunkt ermitteln lässt. Unter der Bedingung, dass aus mindestens zwei Messpositionen die umliegenden Teilbereiche um den Raumwinkel mit der höchsten ermittelten Säulendichte messtechnisch erfassbar sind, wird nach Prüfung von Plausibilitätskriterien für den Gradienten des Signals dieser Schnittpunkt als Punkt der höchsten Konzentration der Gaswolke angenommen.

Das zuvor beschriebene System überwacht bei der Durchführung des beschriebenen Verfahrens vollständig automatisiert große Geländebereiche wie Produktionsstandorte eines Chemieparks oder Verladepunkte wie bspw. Häfen. Dabei ist das System dafür ausgelegt, von geringfügig geschultem Personal bspw. in der Leitstelle einer chemischen Anlage bedient zu werden. Außerdem ist die Lagebewertung des Systems so aufbereitet, dass eine klare, eindeutige Information auf einen Blick erkennbar ist. Diese Information muss für den Anlagenfahrer, den Anlagenbetreiber, die Leitstelle der Werksfeuerwehr und alle Beteiligten eines Krisenstabs gleichermaßen interpretierbar sein. Die Information ist schnell, eindeutig und ohne Vorkenntnisse lesbar, damit das System als ein Frühwarnsystem im Stör- oder Katastrophenfall von Nutzen ist.

Im Folgenden wird die Erfindung anhand von Ausführungsbeispielen mit Bezug auf die Zeichnung erläutert. In der Zeichnung zeigen
- Fig. 1: ein Ausführungsbeispiel eines Systems zur Überwachung eines Luftraumes für ein Gelände,
- Fig. 2a,b,c: Ausführungsbeispiele von optischen Sensoren,
- Fig. 3: eine schematische Darstellung der Raumwinkel zweier optischer Sensoren mit maximalen Säulendichten eines Zielstoffes,
- Fig. 4: die Darstellung nach Fig. 3 zusätzlich mit allen Raumwinkeln mit erhöhter Säulendichte eines Zielstoffes,
- Fig. 5: die Darstellung nach Fig. 4 mit einer teilweisen Abschattung eines Überwachungsbereiches,
- Fig. 6: eine kartographische Darstellung eines Hafens mit einem System mit zwei optischen Sensoren,
- Fig. 7: die Darstellung nach Fig. 6 mit einem lokalisierten Ereignisfall,
- Fig. 8: eine kartographische Darstellung einer Chemieanlage mit einem System von passiven optischen Sensoren und aktiven Messstrecken,
- Fig. 9: eine kartographische Darstellung einer weiteren Chemieanlage mit einer teilweisen Abschattung der Überwachungsbereiche von drei optischen Sensoren,
- Fig. 10: die Darstellung aus Fig. 9 mit einem veränderten Ort der nachgewiesenen Zielstoff-Wolke,
- Fig. 11: die Darstellung aus Fig. 8 mit stationären Sensoren und mit einem mobilen Sensor zum Detektieren einer Zielstoff-Wolke und
- Fig. 12: ein Ausführungsbeispiel eines erfindungsgemäßen Systems zur Überwachung eines Luftraumes für ein Gelände mit einem optischen Sensor und mit einem mobilen nicht-optischen Sensor.

In der nachfolgenden Beschreibung der verschiedenen Ausführungsbeispiele werden Bauteile und Elemente mit gleicher Funktion und gleicher Wirkungsweise mit denselben Bezugszeichen versehen, auch wenn die Bauteile und Elemente bei den verschiedenen Ausführungsbeispielen in ihrer Dimension oder Form Unterschiede aufweisen können.

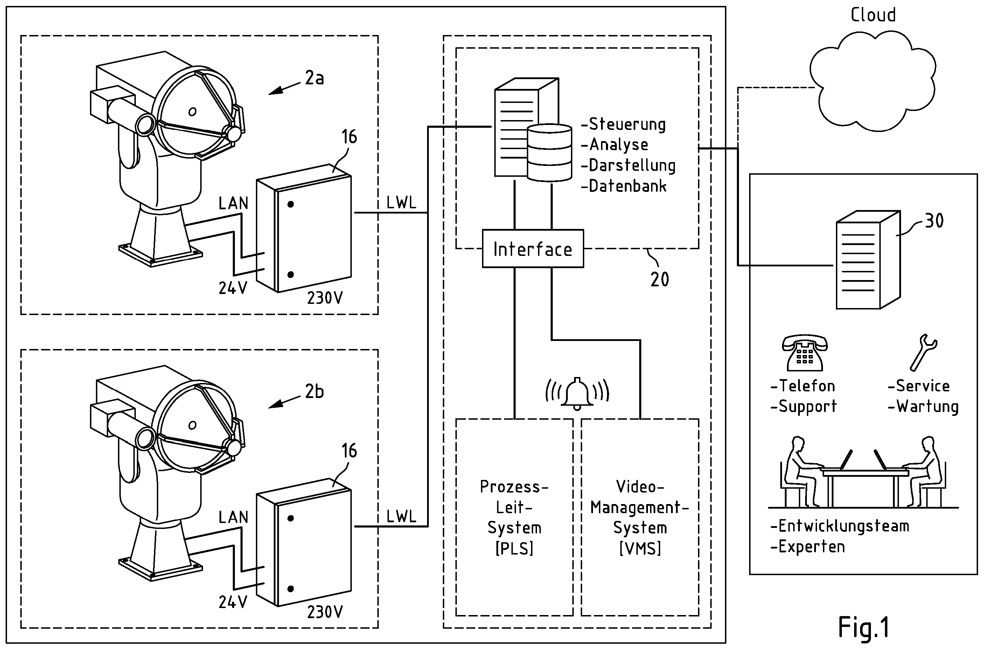

Fig. 1 zeigt einen schematischen Aufbau eines Systems zur Überwachung eines Luftraumes für ein Gelände.

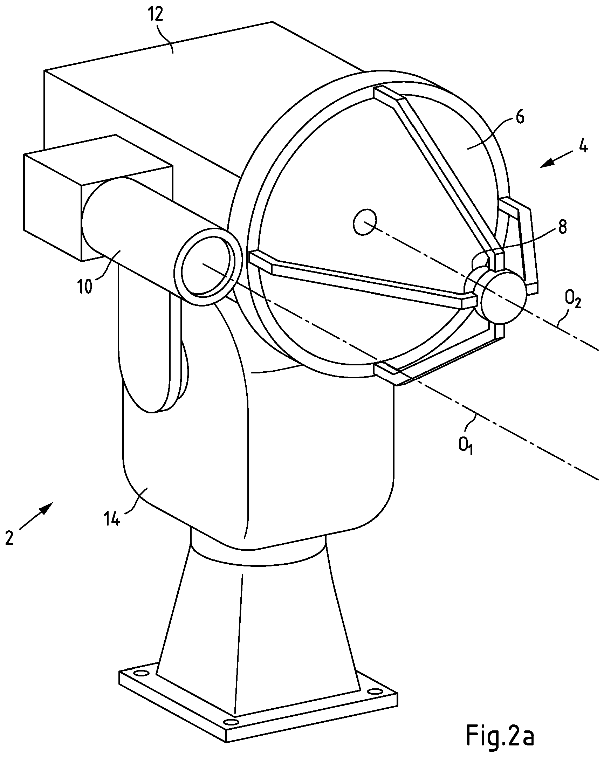

Das System weist zwei optische Sensoren 2a und 2b auf, die in den Fig. 2a bis 2c näher dargestellt sind. Jeder optischer Sensor 2 (bzw. 2a und 2b) weist grundsätzlich eine Infrarotoptik 4 in Form eines IR-Teleskops mit Parabolspiegel 6 und Sekundärspiegel 8, eine Videokamera 10 bzw. 11, ein FTIR-Spektrometer 12 und eine ansteuerbare Schwenk-Neigemimik 14 als Positioniereinheit auf.

Die beiden optischen Sensoren 2a und 2b sind mit einer Versorgungs- und Schnittstelleneinrichtung 16 verbunden, mit der einerseits die Versorgungsspannung von beispielsweise 24 Volt für die optischen Sensoren 2a bzw. 2b bereitgestellt wird. Andererseits findet die Datenübertragung mittels eines Ethernetkabels an ein Local Area Network (LAN) statt. Die Einrichtung 16 kann somit einerseits Steuerdaten auf die optischen Sensoren 2a bzw. 2b übertragen und andererseits die Messdaten empfangen. Alternativ kann vorgesehen sein, dass die Schnittstelleneinrichtung 16 und/oder die Sensoren 2a, 2b die Steuerdaten drahtlos empfangen und/oder die Messdaten drahtlos übertragen.

Über Lichtwellenleiter LWL werden Daten mit einem Server 20 ausgetauscht, in dem Computerprogramme zur Steuerung der optischen Sensoren 2a und 2b, zur Analyse und Darstellung der Daten und dem Speichern insbesondere der Messdaten in einer Datenbank gespeichert sind und ablaufen.

Über eine Schnittstelle (Interface) werden die Ausgabeinformationen an ein Prozessleitsystem und ein Videomanagement weitergeleitet, das sich im Umfeld des überwachten Geländes befindet. Somit kann hier auch im Ereignisfall ein Alarm ausgelöst werden (Glockensymbol) und gleichzeitig können die relevanten Informationen einem Benutzer angezeigt werden.

Des Weiteren kann das System auch eine Übertragung der Daten in eine Cloudanwendung ermöglichen und/oder eine direkte Datenverbindung mit einem externen Server 30 aufweisen. Der externe Server 30 kann dann für unterstützende Funktionen (Telefon, Support, Service, Wartung) und für die Unterstützung eines Entwicklungsteams mit Experten genutzt werden.

Fig. 2a zeigt den oben bereits beschriebenen optischen Sensor 2 zur Überwachung eines Luftraumes für ein Gelände, mit einem FTIR-Spektrometer 12 zur Detektion von Zielstoffen, mit einer Infrarotoptik 4 zur Abbildung eines Teilausschnitts des zu überwachenden Luftraums des Geländes auf das FTIR-Spektrometer 12, mit einer Videokamera 10 und mit einer Positioniereinheit 14 zur Ausrichtung der aus dem FTIR-Spektrometer 12, der Infrarot-Optik 4 und der Kamera 10 gebildeten Sensoreinheit. Dabei erfassen die Infrarot-Optik 4 und die Kamera 10 im Wesentlichen einen gleichen Raumwinkel, insbesondere sind die optische Achse O₁ der Infrarot-Optik und die optische Achse O₂ der Kamera 10 parallel zueinander ausgerichtet.

Die Infrarot-Optik 4 ist im dargestellten Ausführungsbeispiel als Cassegrain-Teleskop mit einem Parabolspiegel 6 und mit einem Sekundärspiegel 8 ausgebildet. Alternativ, aber nicht dargestellt, kann die Infrarot-Optik auch als Linsenoptik ausgebildet sein.

Die Kamera 10 ist seitlich zur Infrarot-Optik 4 angeordnet und hat daher genügend Bauraum, um mit einem Teleobjektiv ausgerüstet zu sein, um somit eine hohe Bildqualität des überwachten Teilausschnitts des Luftraums des Geländes zu erzeugen. Allerdings muss für eine genaue Überlagerung der Messdaten von Zielstoff-Wolken die Parallaxe durch den Abstand der optischen Achsen ausgeglichen werden.

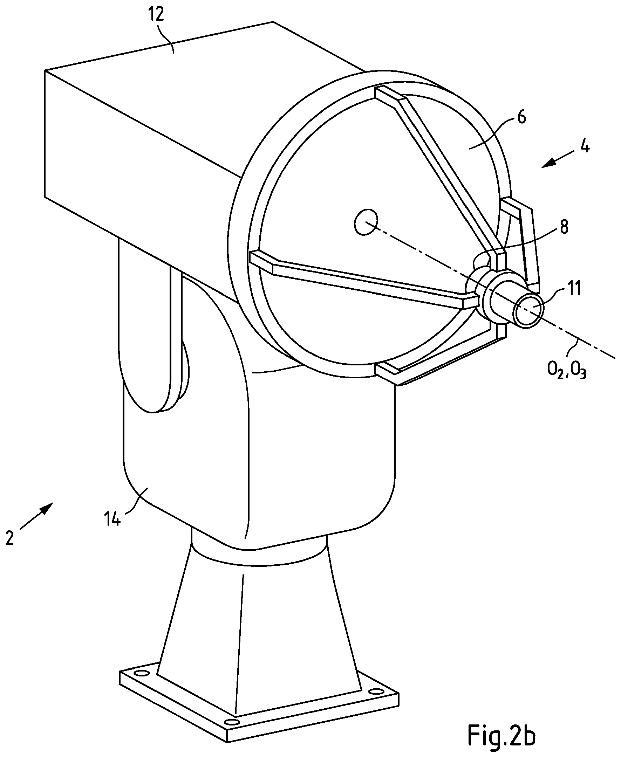

Fig. 2b zeigt ein weiteres Ausführungsbeispiel eines optischen Sensors 2. Im Unterschied zu Fig. 2a ist eine Kamera 11 in der optischen Achse der Infrarot-Optik 4 am vorderen Ende des Sekundärspiegels angeordnet. Somit fallen die optische Achse O₂ der Infrarot-Optik 4 und die optische Achse O₃ der Kamera 11 zusammen und ein Parallaxen-Effekt wird vermieden. Durch die geringe Baugröße der Kamera 11, die beispielsweise eine Smartphone-Kamera sein kann, ist der Beobachtungswinkel in der Regel groß, so dass die Beobachtungswinkel größer und die räumliche Auflösung des Kamerabildes kleiner als bei einer Kamera mit Teleobjektiv sein können. Jedoch sind auch schon Smartphone-Kameras mit einer Telefunktion bekannt, so dass die Kamera 10 vollständig ersetzt werden kann.

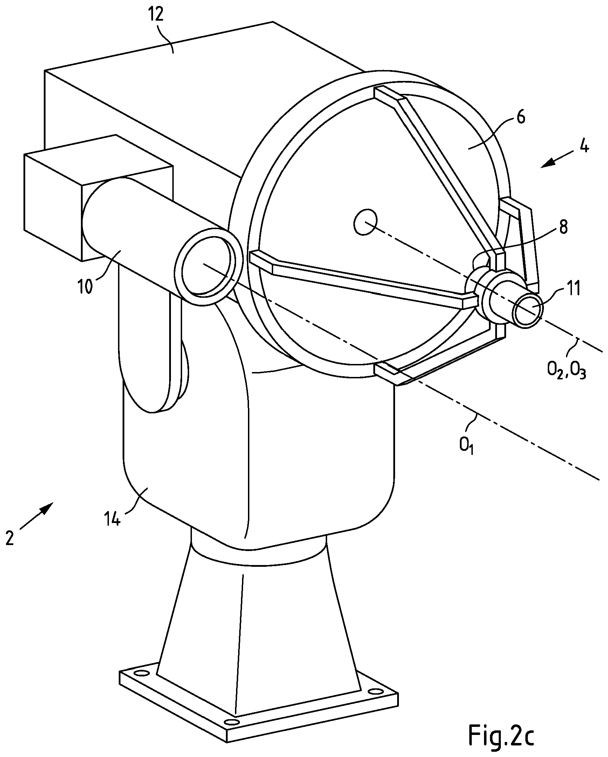

Fig. 2c zeigt ein weiteres Ausführungsbeispiel eines optischen Sensors 2. Im Unterschied zu den Fig. 2a und 2b ist die Kamera als Kamerasystem mit einer seitlich zur Infrarot-Optik 4 angeordneten Kamera 10 und mit einer in der optischen Achse O₂ der Infrarot-Optik 4 angeordneten Kamera 11 ausgebildet. Somit verläuft die optische Achse O₁ der Kamera 10 beabstandet zur optischen Achse O₂ der Infrarot-Optik 4, wobei die optische Achse O₃ der Kamera 11 mit der optischen Achse O₂ zusammenfällt und somit keinen oder nur einen geringen Abstand davon aufweist.

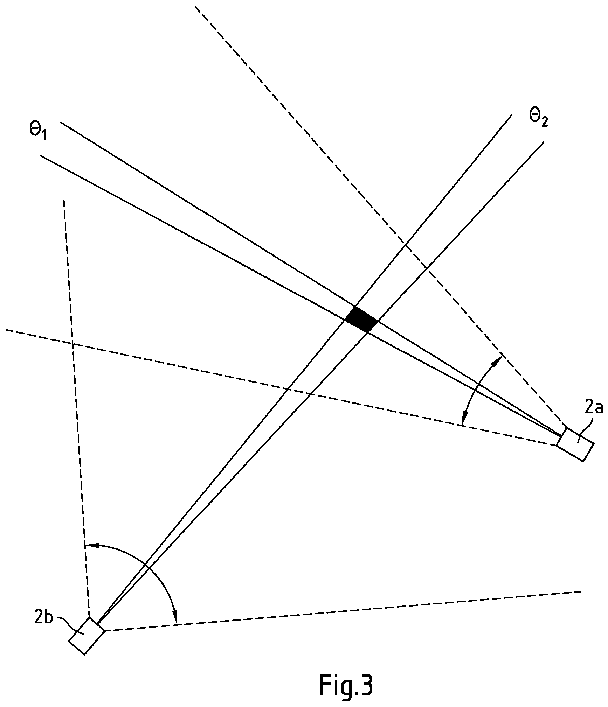

Fig. 3 zeigt eine schematische Darstellung der Überwachung durch zwei optische Sensoren 2a und 2b. Der erste optische Sensor 2a weist einen Überwachungsbereich auf, der mit gestrichelten Linien eingezeichnet ist und ca. 35° breit ist. Den Überwachungsbereich scannt der optische Sensor 2a im Regelfall zumindest teilweise entlang eines vorgegebenen Pfades ab. Der zweite optische Sensor 2b weist einen Überwachungsbereich von ca. 90° auf, wie ebenfalls mit gestrichelten Linien dargestellt ist. Auch der zweite optische Sensor 2b scannt automatisch den Überwachungsbereich zumindest teilweise entlang eines voreingestellten Pfades ab. Vorzugsweise wird das Scannen zyklisch wiederholt. Die Überwachungsbereiche beider optischen Sensoren überlappen sich. Während der Messungen werden die Daten auf den Server 30 übertragen und mit den Positionsdaten (Raumwinkel) kombiniert.

Aus den Messdaten der optischen Sensoren 2a und 2b wird für jeden Raumwinkel die spektrale Intensitätsverteilung der empfangenen IR-Strahlung abgeleitet, um mittels Korrelation der Intensitätsverteilung mit bekannten Gasspektren mindestens einen Zielstoff zu identifizieren. Dazu läuft ein entsprechendes Computerprogramm auf dem Server 30 oder gegebenenfalls in dem optischen Sensor 2a bzw. 2b ab.

Im Ereignisfall, also wenn der erste optische Sensor 2a einen Zielstoff, also ein Gas der Zielstoffliste in einem Raumwinkel Θ₁ identifiziert, wird der weitere optische Sensor 2b angesteuert, den Überlappungsbereich mit dem Überwachungsbereich des ersten optischen Sensors zu scannen.

Aus den Messdaten des optischen Sensors 2b wird ein weiterer Raumwinkel Θ₂ mit einem Infrarotsignal des Zielstoffes identifiziert, so dass aus den Raumwinkelinformationen des ersten Raumwinkels Θ₁ und des weiteren Raumwinkels Θ₂ die Koordinaten des Überlappungsbereiches (schwarze Fläche) mit erhöhter Konzentration des Zielstoffes ermittelt werden können.

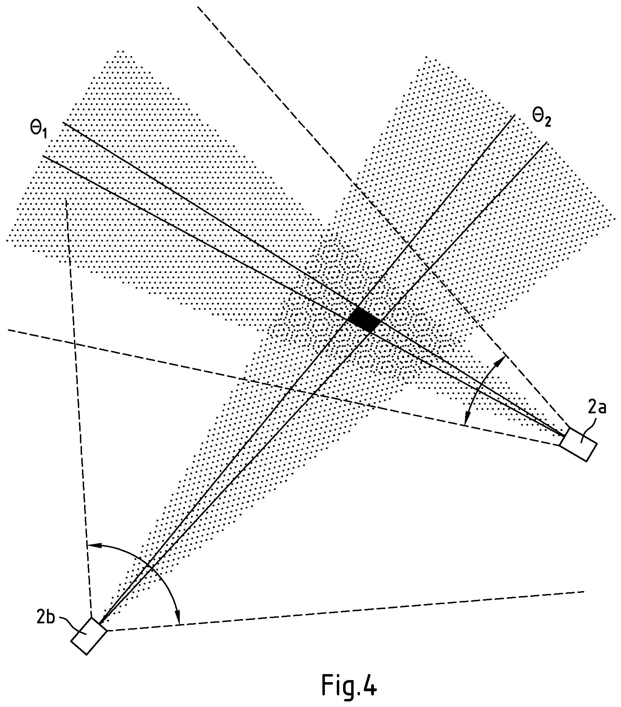

Fig. 4 zeigt eine erweiterte Darstellung zu Fig. 3, bei der die gepunkteten Bereiche der Überwachungsbereiche der optischen Sensoren 2a und 2b die Raumwinkelbereiche darstellen, in denen jeweils zumindest eine geringe Konzentration des Zielstoffes identifiziert worden ist. Die bereits in Fig. 3 dargestellten Raumwinkelbereiche Θ₁ und Θ₂ zeigen die Raumwinkelbereiche mit höchster Konzentration des Zielstoffes, die durch Berechnung der Säulendichte ermittelt worden ist. Zur Berechnung der Säulendichte siehe oben die allgemeine Beschreibung. Nach Fig. 4 kann somit nicht nur das Zentrum der Gaswolke des Zielstoffes (schwarzes Feld), sondern auch die Ausdehnung der Wolke bestimmt werden.

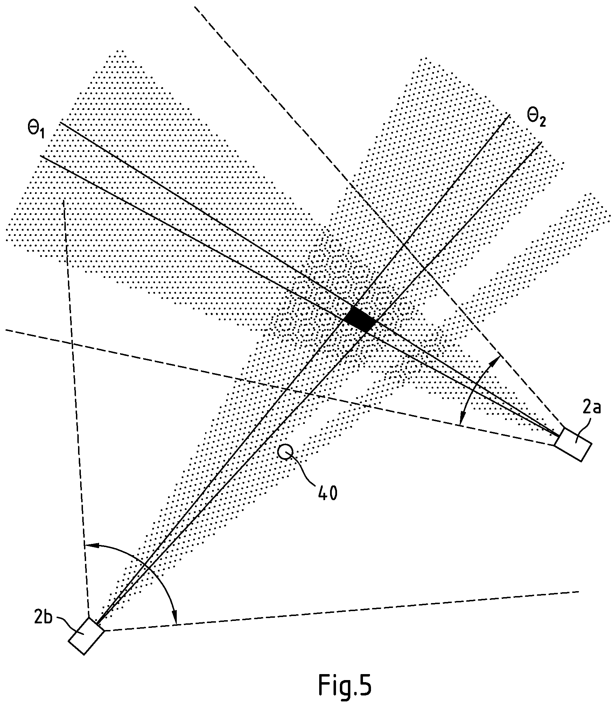

Fig. 5 zeigt eine ähnliche Abbildung wie in Fig. 4. Hier ist im Überwachungsbereich des zweiten optischen Sensors 2b ein Turm 40 auf dem überwachten Gelände angeordnet, so dass der von dem optischen Sensor 2b hinter dem Turm 40 angeordnete Bereich abgeschattet wird. Der abgeschattete Bereich kann somit von dem optischen Sensor 2b nicht überwacht werden, der Messradius des optischen Sensors 2b reicht in diesem Bereich nur bis zum Turm 40 und somit nicht bis zum Überwachungsbereich des optischen Sensors 2a. Aus diesem Grund ist der "Schatten" auch nicht gepunktet in Fig. 5 dargestellt.

Bei der Suche der genauen Lokalisation der Wolke aus dem Zielstoff werden die Messsignale des optischen Sensors 2b in Raumrichtungen mit zu geringem Messradius nicht in die Auswertung einbezogen. Hier sind für die von dem Turm 40 abgedeckten Raumwinkel die Messradien zu gering und die Überwachungsbereiche der Sensoren 2a, 2b überlappen sich in einer hinter dem Turm 40 liegenden Abschattung nicht. Trotzdem kann die Ausdehnung der Wolke des Zielstoffes nahezu vollständig erfasst werden. Durch die Nichtberücksichtigung der Messdaten in Raumrichtungen mit zu geringem Messradius wird die Auswertung nicht verfälscht, denn in dem Überwachungsbereich, der beim Turm 40 endet, wird das Messsignal keinen Hinweis auf den Zielstoff ergeben, obwohl dieses ohne Anwesenheit des Turms 40 der Fall wäre, siehe Fig. 4.

Gegebenenfalls kann der Server 20 eingerichtet sein, also durch Anwendung von Computerprogrammen, die Messsignale des weiteren optischen Sensors 2b, die nicht in die Auswertung einbezogen worden sind, durch mathematische Interpolation von angrenzenden Messsignalen des optischen Sensors 2b zu ersetzen. Angrenzende Messsignale sind dabei solche, deren zugeordnete Raumwinkelbereiche benachbart zu den Raumwinkelbereichen der nicht einbezogenen Messsignale angeordnet sind.

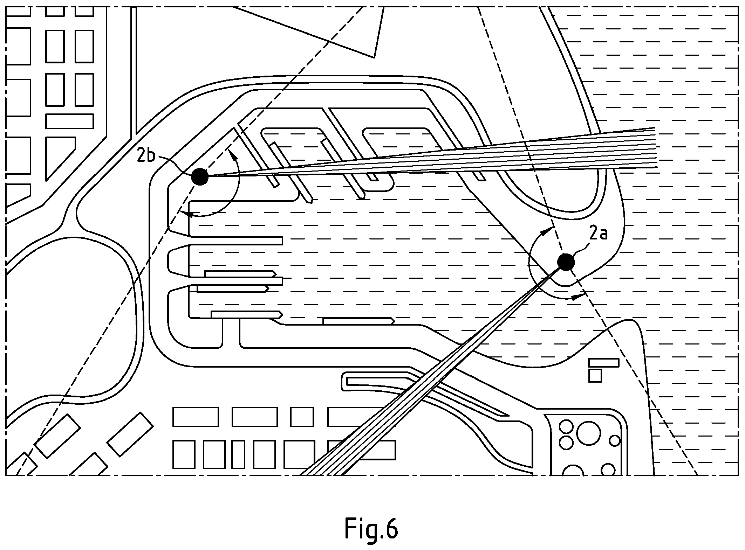

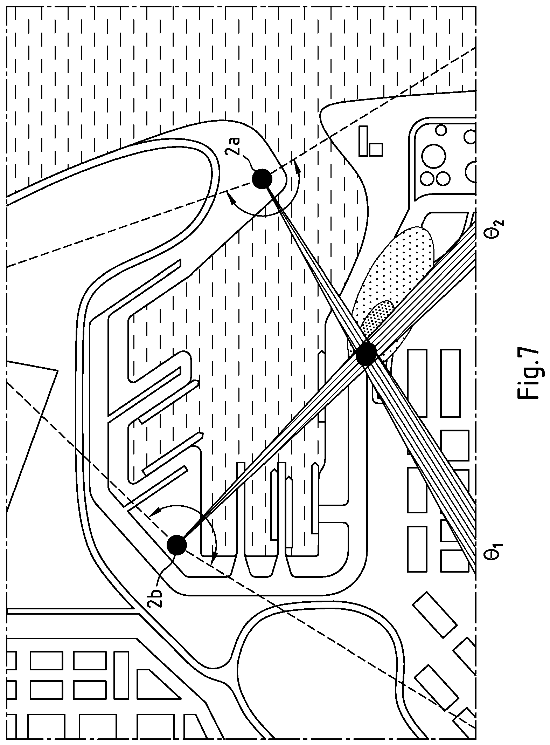

Die Fig. 6 und 7 zeigen ein System zur Überwachung eines Luftraumes für ein Gelände eines Hafenbeckens, bei dem die beiden optischen Sensoren 2a und 2b an prominenten Positionen innerhalb des Hafengeländes positioniert sind.

In Fig. 6 ist der Regelfall dargestellt, bei dem die beiden optischen Sensoren 2a und 2b unabhängig voneinander den Luftraum oberhalb des Geländes innerhalb der mit gestrichelten Linien gekennzeichneten Überwachungsbereichen scannen.

Fig. 7 zeigt den Ereignisfalls, wie er zuvor schon beschrieben worden ist. Der Server 20 ist eingerichtet durch Anwendung eines Computerprogrammes, die Koordinaten des Überlappungsbereiches (schwarze Fläche in Fig. 7) der Raumwinkelbereiche Θ₁ und Θ₂ der höchsten Säulendichten der beiden optischen Sensoren 2a und 2b zu ermitteln und die Koordinaten mit einer Kartendarstellung zu verknüpfen und eine zweidimensionale Darstellung des Ereignisses zu erstellen. Vorzugsweise kann auch durch Verknüpfen mit Bildern der Kameras 11 der Sensoren 2a, 2b eine dreidimensionale Darstellung erstellt werden.

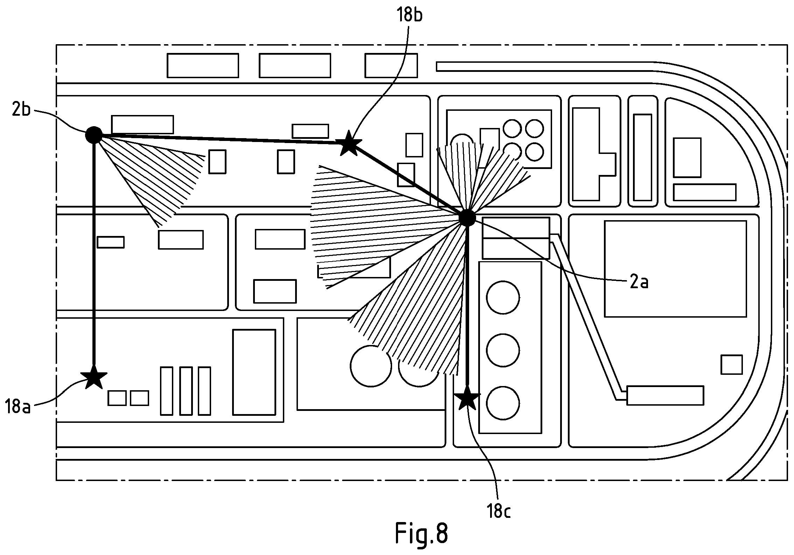

Fig. 8 zeigt ein weiteres System zur Überwachung eines Luftraumes für ein ausgedehntes Gelände einer Chemieproduktion. Erneut sind zwei optische Sensoren 2a und 2b an prominenten Positionen angeordnet. Zusätzlich sind drei aktive Infrarotstrahlungsquellen 18a, 18b und 18c so angeordnet, wobei jeweils eine der optischen Sensoren 2a oder 2b das Infrarotlicht aufnimmt. Dadurch wird entlang der eingerichteten Messstrecken (dicke Striche in Fig. 8) eine genauere Messung der in der Atmosphäre enthaltenen Gase möglich. Des Weiteren kann auch der Spektralbereich zur Auswertung verbreitert werden, was eine größere Anzahl an zu messenden Zielstoffen ermöglicht. Die aktiven Messungen ermöglichen unter anderem eine genauere Hintergrundbestimmung der Gasverteilung sowie eine größere Zielstoffbibliothek. Ferner ermöglichen die aktiven Infrarotstrahlungsquellen 18a, 18b, 18c ein gesondertes Überwachen vordefinierter Grenzbereiche, wie beispielsweise Anlagengrenzen.

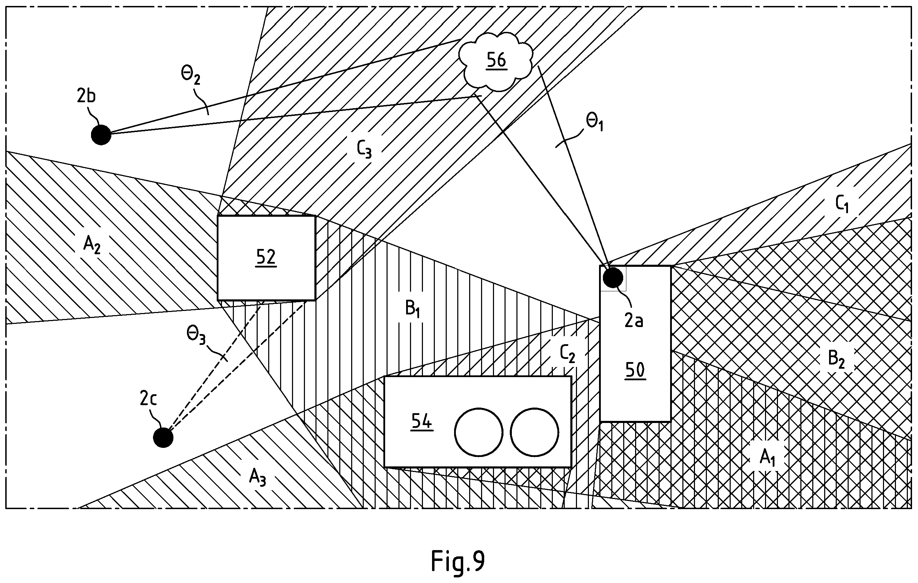

Fig. 9 zeigt eine kartographische Darstellung einer weiteren Chemieanlage mit einer teilweisen Abschattung der Überwachungsbereiche von drei optischen Sensoren 2a, 2b und 2c.

Auf dem Gelände sind Gebäude 50, 52 und 54 vorhanden, die den Überwachungsbereich des optischen Sensors 2a teilweise abschatten, so dass sich Schattenbereiche A₁, A₂ und A₃ ergeben. Der Überwachungsbereich des optischen Sensors 2a erstreckt sich daher in den zugeordneten Raumwinkeln jeweils nur bis zum Gebäude 50, 52 bzw. 54.

In gleicher Weise ergeben sich für den optischen Sensor 2b Abschattungsbereiche B₁ und B₂ und für den optischen Sensor 2c Abschattungsbereiche C₁, C₂ und C₃.

Fig. 9 zeigt das System mit den drei optischen Sensoren 2a, 2b und 2c mit einer Zielstoff-Wolke 56. Die Zielstoff-Wolke 56 wird im Regelfall der scannenden optischen Sensoren 2a, 2b und 2c als Erstes durch den Sensor 2a detektiert und ein Ereignisfall wird festgestellt.

Der in dieser Figur nicht dargestellte Server ist eingerichtet, in diesem Ereignisfall zumindest einen weiteren optischen Sensor 2b für eine Ansteuerung auszuwählen, dessen Überwachungsbereich eine maximale Überlappung mit dem Überwachungsbereich des ersten optischen Sensors 2a aufweist. Die Abschattungen B₁ und B₂ liegen außerhalb des Raumwinkelbereichs Θ₁ des ersten optischen Sensors 2a, so dass eine maximale Überlappung durch den Überwachungsbereich des Sensors 2b gegeben ist. Der zweite Sensor 2b detektiert dann die Zielstoff-Wolke 56 innerhalb des Raumwinkelbereichs Θ₂ und die Zielstoff-Wolke 56 wird lokalisiert.

Der dritte Sensor 2c wird in diesem Szenario nicht ausgewählt, weil der Abschattungsbereich C₃ einen Teil des Raumwinkelbereichs Θ₁ überlappt. Eine Auswahl des optischen Sensors 2c hätte dann auch nicht zu einer positiven Messung der Zielstoff-Wolke 56 geführt, weil die Zielstoff-Wolke 56 vollständig in dem Abschattungsbereich C₃ liegt. Der Messradius des dritten Sensors 2c ist hier also in dem Raumwinkelbereich Θ₃ zu gering. Der potentielle Raumwinkel Θ₃ ist nur gestrichelt eingezeichnet, da die Abschattung durch das Gebäude 52 eine Messung der Zielstoff-Wolke 56 verhindert.

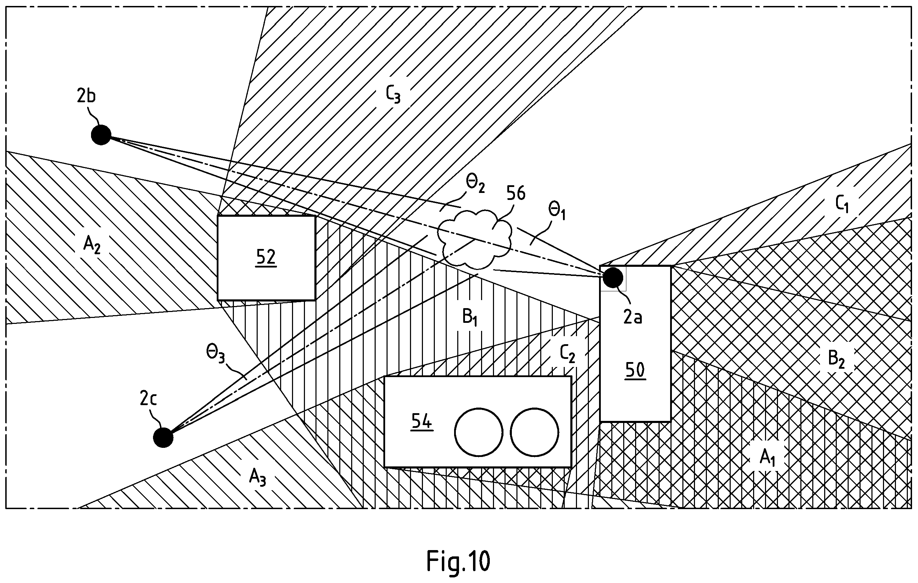

Fig. 10 zeigt die Darstellung aus Fig. 9 mit einem veränderten Ort der nachgewiesenen Zielstoff-Wolke 56.

Nachdem der optische Sensor 2a die Zielstoff-Wolke detektiert hat, wird vom Server ein weiterer optischer Sensor gesucht, um die Lokalisation der Zielstoff-Wolke 56 zu erzielen. Dabei wird festgestellt, dass beide optische Sensoren 2b und 2c jeweils einen Abschattungsbereich aufweisen, der mit dem Raumwinkelbereich Θ₁ überlappt. Der optische Sensor 2b wird aber vom Server nicht ausgewählt, weil die Position des optischen Sensors 2b in Richtung des Raumwinkelbereichs Θ₁ des ersten optischen Sensors 2a mit nachgewiesenem Zielstoff liegt. Daher wird der dritte optische Sensor 2c ausgewählt, dessen Überwachungsbereich Raumwinkel mit einem größeren Winkel zum gemessenen Raumwinkelbereich Θ₁ des ersten optischen Sensors 2a aufweist, um die Zielstoff-Wolke 56 zu identifizieren, den Raumwinkelbereich Θ₃ und somit die Koordinaten der Zielstoff-Wolke 56 zu bestimmen.

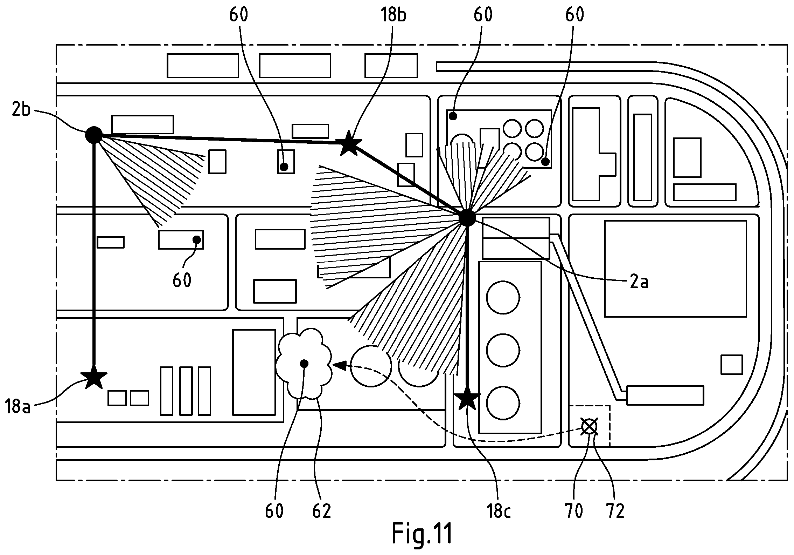

Fig. 11 zeigt ein weiteres Ausführungsbeispiel des zuvor beschriebenen Systems zur Überwachung eines Luftraumes für ein Gelände, das beispielhaft auf der Ausgestaltung nach Fig. 8 aufbaut.

Zusätzlich zu der oben beschriebenen Ausgestaltung des Systems ist hier zusätzlich vorgesehen, dass stationäre Detektoren 60, die als chemo-elektrische Detektoren ausgebildet sind. Der Server (hier nicht dargestellt) ist eingerichtet, ein Ausgangssignal des mindestens einen stationären Detektors 60 als auslösendes Signal für den Einsatz der optischen Sensoren 2a, 2b im Raumbereich des stationären Detektors zu verwenden. Dargestellt ist eine Mehrzahl von Detektoren 60, die sämtlich in Raumwinkelbereichen positioniert sind, die von den optischen Sensoren 2a und 2b erfasst werden können. Auch wenn hier mehrere Detektoren 60 dargestellt sind, ist es ausreichend, wenn nur ein Detektor 60 vorhanden ist.

Wenn ein Ereignisfall mit einem Austritt eines Zielstoffes auftritt und sich eine Wolke 62 ausbreitet, dann kann der in der Wolke 62 angeordnete Detektor 60 den Zielstoff detektieren und ein entsprechendes Signal an den Server senden. Anschließend können dann die beiden optischen Sensoren 2a und 2b, sofern diese noch nicht den Ereignisfall erfasst haben sollten, die Gaswolke 62 erfassen und wie zuvor beschrieben verarbeiten.

Wie in Fig. 11 auch zu erkennen ist, kann mindestens ein mobiler Sensor 70 montiert an einer Drohne 72 ausgebildet sein. Dabei kann der mobile Sensor 70 als optischer Sensor 2 oder als Detektor 60 ausgebildet sein. Wie mit einer gestrichelten Linie dargestellt ist, wird der mobile Sensor 70 im Ereignisfall zur Wolke 62 geführt und kann Vorort zusätzliche Messungen machen, die vom System und dem Server ausgewertet werden können.

Wenn der mobile Sensor 70 einen optischen Sensor 2 aufweist, dann kann das Messsignal des mobilen Sensors 70 zusammen mit den Messsignalen der anderen stationären optischen Sensoren 2a und 2b ausgewertet werden, wie es zuvor beschrieben worden ist.

Wenn der mobile Sensor 70 einen Detektor 60 aufweist, dann können durch die Variabilität der Positionen des mobilen Sensors 70 zusätzlich zu den Messwerten des stationären Detektors 60 weiteren Daten erfasst werden.

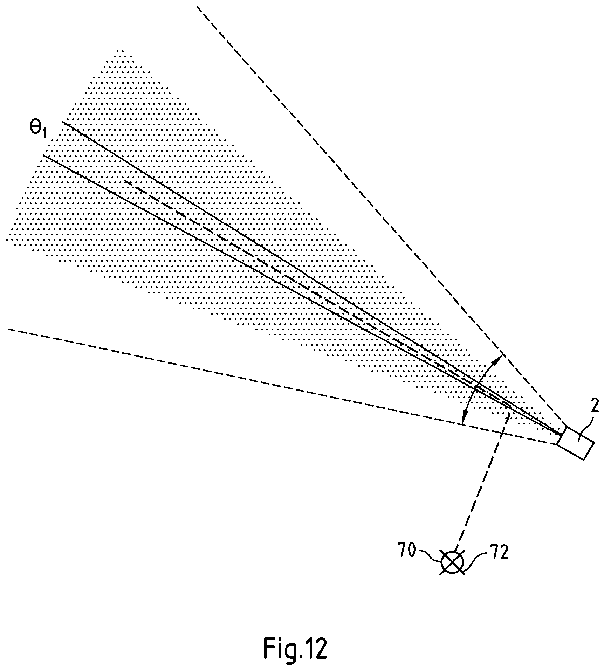

Fig. 12 zeigt ein Ausführungsbeispiel eines erfindungsgemäßen Systems zur Überwachung eines Luftraumes für ein Gelände. Dieses System entspricht im Wesentlichen dem System nach Fig. 4, wobei jedoch nur ein optischer Sensor 2 mit einem passiven Fourier-Transform-Infrarotspektrometer vorhanden ist. Wie oben beschrieben ist ein Server (nicht dargestellt) zur Auswertung der Messdaten und zur Steuerung des optischen Sensors 2 vorgesehen. Der optische Sensor 2 weist - wie beschrieben - einen einstellbaren Überwachungsbereich auf.

Ein mobiler flugfähiger Sensor 70 der zuvor beschriebenen Art ist vorgesehen, der an einer zuvor beschriebenen Drohne 72 befestigt ist. Im Ereignisfall, wenn also der optische Sensor 2 in einem Raumwinkel (gepunktet dargestellt) einen Zielstoff identifiziert, dessen Säulendichte innerhalb des Winkels Θ₁ am höchsten ist, wird der mobile Sensor 70 vom Server angesteuert, die Konzentration des Zielstoffes entlang des vom optischen Sensor 2 identifizierten Raumwinkels ortsabhängig zu erfassen. Die entsprechend Flugroute ist in Fig. 12 gestrichelt dargestellt.

Somit kann mit nur einem optischen Sensor 2 und einem mobilen Sensor 70 der Ort der maximalen Konzentration des Zielstoffes, als schwarze Fläche dargestellt, ermittelt werden.

## Patentansprüche

1. System zur Überwachung eines Luftraumes für ein Gelände,
- mit mindestens einem optischen Sensor (2; 2a, 2b, 2c) mit einem passiven Fourier-Transform-Infrarotspektrometer und
- mit einem Server (20, 30) zur Auswertung der Messdaten und zur Steuerung des mindestens einen optischen Sensors (2; 2a, 2b, 2c),
- wobei der mindestens eine optische Sensor (2; 2a, 2b, 2c) einen einstellbaren Überwachungsbereich aufweist, wobei das System mindestens einen mobilen flugfähigen Detektor (70) umfasst und
- der Server (20, 30) eingerichtet ist,
-- den optischen Sensor (2; 2a, 2b, 2c) im Regelfall zu einem automatischen Scannen der Überwachungsbereiche anzusteuern, wobei der Server den Messdaten jeweils einen Raumwinkel anhand der Positionsdaten des optischen Sensors (2; 2a, 2b, 2c) zuordnet,
-- aus den Messdaten des optischen Sensors (2; 2a, 2b, 2c) für jeden Raumwinkel die spektrale Intensitätsverteilung der empfangenen IR-Strahlung abzuleiten und mittels Korrelation der Intensitätsverteilung mit bekannten Gasspektren mindestens einen Zielstoff zu identifizieren und
-- im Ereignisfall, wenn der optische Sensor (2; 2a, 2b, 2c) in einem Raumwinkel einen Zielstoff identifiziert, mit dem mindestens einen mobilen flugfähigen Detektor (70) die Konzentration des Zielstoffes entlang des vom optischen Sensor (2; 2a, 2b, 2c) identifizierten Raumwinkels ortsabhängig zu erfassen.

2. System nach Anspruch 1, wobei der Server (20, 30) lokalisiert den Zielstoff im Ereignisfall durch Ermitteln derjenigen Position des mobilen Sensors im Überwachungsbereich des ersten Sensors, für die die Konzentration des Zielstoffs maximal ist.

## Claims

1. System for monitoring an airspace for an area,
- with at least one optical sensor (2; 2a, 2b, 2c) with a passive Fourier transform infrared spectrometer and
- with a server (20, 30) for evaluating the measurement data and for controlling the at least one optical sensor (2; 2a, 2b, 2c),
- wherein the at least one optical sensor (2; 2a, 2b, 2c) has an adjustable monitoring area,
- wherein the system comprises at least one mobile airworthy detector (70) and the server (20, 30) is set up,
- as a general rule, to control the optical sensor (2; 2a, 2b, 2c) to automatically scan the monitoring areas, wherein the server assigns a solid angle to the measurement data in each case on the basis of the position data of the optical sensor (2; 2a, 2b, 2c),
- to derive the spectral intensity distribution of the received IR radiation for each solid angle from the measurement data of the optical sensor (2; 2a, 2b, 2c) and identifying at least one target substance by means of correlation of the intensity distribution with known gas spectra, and
- in case of an incident, when the optical sensor (2; 2a, 2b, 2c) identifies a target substance in a solid angle, to detect the concentration of the target substance along the solid angle identified by the optical sensor (2; 2a, 2b, 2c) with the at least one mobile airworthy detector (70) in a location-dependent manner.

2. System according to claim 1,
wherein the server (20, 30) locates the target substance in case of an incident by determining that position of the mobile sensor in the monitoring area of the first sensor for which the concentration of the target substance is maximum.

## Revendications

1. Système de surveillance d'un espace aérien pour un terrain,
- avec au moins un capteur optique (2 ; 2a, 2b, 2c) comprenant un spectromètre infrarouge à transformée de Fourier passif et
- avec un serveur (20, 30) permettant d'évaluer les données de mesure et permettant de commander le au moins un capteur optique (2 ; 2a, 2b, 2c),
- dans lequel le au moins un capteur optique (2 ; 2a, 2b, 2c) présente une région de surveillance ajustable,
dans lequel le système comprend au moins un détecteur mobile capable de voler (70) et
- le serveur (20, 30) est conçu pour,
-- commander le capteur optique (2 ; 2a, 2b, 2c) en règle générale pour un balayage automatique des régions de surveillance, dans lequel le serveur associe aux données de mesure respectivement un angle spatial en se basant sur les données de position du capteur optique (2 ; 2a, 2b, 2c),
-- à partir des données de mesure du capteur optique (2 ; 2a, 2b, 2c), déduire pour chaque angle spatial la distribution d'intensité spectrale du rayonnement IR reçu et identifier au moins une substance cible au moyen d'une corrélation entre la distribution d'intensité et des spectres de gaz connus et, en cas d'événement, lorsque le capteur optique (2 ; 2a, 2b, 2c) identifie une substance cible dans un angle spatial, détecter avec le au moins un détecteur mobile capable de voler (70) la concentration de la substance cible en fonction de son emplacement le long de l'angle spatial identifié par le capteur optique (2 ; 2a, 2b, 2c).

2. Système selon la revendication 1,
dans lequel le serveur (20, 30) localise la substance cible en cas d'événement grâce à une étape consistant à déterminer la position du capteur mobile pour laquelle la concentration de la substance cible est maximale dans la région de surveillance du premier capteur.
